(19) **Europäisches Patentamt**
**European Patent Office**
**Office européen des brevets**

(11) **EP 4 543 589 B1**

(12) **FASCICULE DE BREVET EUROPEEN**

(45) Date de publication et mention de la délivrance du brevet:
**25.02.2026 Bulletin 2026/09**

(21) Numéro de dépôt: **23732976.8**

(22) Date de dépôt: **16.06.2023**

(51) Classification Internationale des Brevets (IPC):
**G01N 15/1031** (2024.01)  **G01N 27/74** (2006.01)
**G01N 15/01** (2024.01)  **G01N 15/10** (2024.01)
**G01N 33/543** (2006.01)

(52) Classification Coopérative des Brevets (CPC):
**G01N 27/745; G01N 15/1031;** B01L 2200/0652; B01L 2300/0663; B01L 2400/043; G01N 15/01; G01N 33/54326; G01N 2015/1024

(86) Numéro de dépôt international:
**PCT/EP2023/066292**

(87) Numéro de publication internationale:
**WO 2023/247370 (28.12.2023 Gazette 2023/52)**

(54) **PROCÉDÉ DE DÉTECTION MAGNÉTIQUE D'OBJETS BIOLOGIQUES MICROSCOPIQUES ET DISPOSITIFS ASSOCIÉS**

VERFAHREN ZUR MAGNETISCHEN DETEKTION MIKROSKOPISCHER BIOLOGISCHER OBJEKTE UND ZUGEHÖRIGE VORRICHTUNGEN

METHOD FOR MAGNETICALLY DETECTING MICROSCOPIC BIOLOGICAL OBJECTS AND ASSOCIATED DEVICES

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **24.06.2022 FR 2206315**

(43) Date de publication de la demande:
**30.04.2025 Bulletin 2025/18**

(73) Titulaire: **Commissariat à l'Energie Atomique et aux Energies Alternatives**
**75015 Paris (FR)**

(72) Inventeurs:
• **BONVILLE, Pierre**
  **78000 Versailles (FR)**
• **JECKELMANN, Mathieu**
  **1700 Fribourg (CH)**
• **JASMIN-LEBRAS, Guénaëlle**
  **91191 Gif-sur-Yvette (FR)**
• **DEROO, Maïkane**
  **91191 Gif-sur-Yvette (FR)**
• **FERAUDET, Cécile**
  **91191 Gif-sur-Yvette (FR)**

(74) Mandataire: **Cabinet Camus Lebkiri**
**25 rue de Maubeuge**
**75009 Paris (FR)**

(56) Documents cités:
**WO-A1-2019/238857    US-A1- 2019 317 167**

• **ZHU CHENG ET AL: "A micro-array bio detection system based on a GMR sensor with 50-ppm sensitivity", SCIENCE CHINA INFORMATION SCIENCES, SCIENCE CHINA PRESS, BEIJING, vol. 60, no. 8, 24 April 2017 (2017-04-24), pages 1 - 9, XP036227520, ISSN: 1674-733X, [retrieved on 20170424], DOI: 10.1007/S11432-016-0645-2**

**Description**

**DOMAINE TECHNIQUE DE L'INVENTION**

Le domaine technique de l'invention est l'identification d'objets biologiques microscopiques à partir de leur moment magnétique dans le but de développer un dispositif de diagnostic précoce et sensible.

**ARRIÈRE-PLAN TECHNOLOGIQUE DE L'INVENTION**

Le développement de méthodes et de dispositifs de diagnostic dits « précoces », à la fois rapides, sensibles, transportables au chevet du patient et peu coûteux est un vrai défi dans le domaine de la santé mais également dans celui de la défense ou de l'environnement.

Parmi les méthodes de diagnostic précoce, faciles d'utilisation, on trouve la migration de cibles dans de la cellulose, dites « bandelettes ». La méthode de diagnostic mettant en œuvre des bandelettes permet de fournir des résultats en 30 minutes mais a pour inconvénient de ne pas être adaptée à des cibles de grande taille (comme les cellules et certaines bactéries) et surtout de ne pas être suffisamment sensible. D'autres méthodes de diagnostic utilisées dans des laboratoires de biologie comprennent la méthode « ELISA » (pour « enzyme-linked immunosorbent assay » en anglais) ou la méthode « PCR » (pour « Polymerase Chain Reaction » en anglais). Ces méthodes présentent de meilleures sensibilités (comptage compris entre $10^3$ UFC/ml et $10^4$ UFC/ml). En revanche, elles présentent des temps de réalisation variant entre 3 h et 4 h et nécessitent un personnel formé à son utilisation complexe.

Un dispositif couramment utilisé pour la détection d'objets biologiques dans les laboratoires de biologie est un cytomètre de flux. Il permet de dénombrer, un à un, des objets biologiques. Il n'est en revanche pas transportable et est également complexe à utiliser. Il présente également un coût élevé.

Les laboratoires sur puces, dits « lab on chip » en anglais, ou « biopuces », apportent une solution aux problèmes d'encombrement et de complexité. Pour cela, certaines biopuces utilisent la détection optique de cibles. Cependant, ces biopuces restent peu adaptées à l'étude de certaines matrices opaques. D'autres biopuces utilisent la détection électrochimique de cibles. Toutefois, ces biopuces présentent l'inconvénient d'avoir de trop nombreuses interactions non spécifiques avec l'environnement extérieur ou certaines matrices, ce qui réduit la sensibilité de la détection.

Des biopuces, mettant en œuvre la détection magnétique au moyen de capteurs magnétorésistifs, ont connu un fort développement. Les objets biologiques à détecter sont marqués (également dits « labélisés ») au moyen de particules (ou billes) magnétiques fonctionnalisées par des anticorps spécifiques de la cible d'intérêt. Les biopuces à base de capteurs magnétorésistifs détectent ensuite, un par un, en fonction du temps, les objets magnétiques circulant dans un canal microfluidique. La détection peut être réalisée dans des matrices liquides et les échantillons étudiés ne nécessitent pas de lavage préalable. Ils peuvent également être faiblement concentrés. Enfin, le comptage et l'analyse des signaux peuvent être réalisés simultanément.

Parmi les méthodes de détection magnétique développées ces dernières années, les capteurs magnétorésistifs à magnétorésistance géante (dite « GMR ») ou magnétorésistance tunnel (dite « TMR ») sont les seuls à combiner, à la fois, une grande sensibilité de détection, un encombrement réduit et une production industrielle mature. Ces capteurs présentent également un coût de fabrication et d'utilisation bas.

Une biopuce peut comprendre un capteur magnétorésistif GMR ou TMR disposé sous le canal microfluidique. Des objets biologiques labélisés circulent dans ledit canal microfluidique. Le champ dipolaire émis par chaque objet biologique marqué passant à proximité dudit capteur magnétorésistif est mesuré. Quand le champ dipolaire émis est supérieur à une limite de détection du capteur GMR, le signal est comptabilisé.

Cette façon de procéder comprend toutefois quelques inconvénients majeurs. En effet le champ dipolaire est proportionnel à $\mu/z^3$, où $\mu$ est le moment magnétique de l'objet magnétique détecté et $z_b$ sa hauteur de passage dans le canal au-dessus du capteur. Ainsi un signal provenant d'un petit agrégat de billes magnétiques (par exemple, un agrégat sans aucun objet biologique) passant près du capteur GMR ($\mu$ et z faibles) peut donner le même signal qu'un objet biologique correctement marqué magnétiquement mais passant plus haut dans le canal ($\mu$ et z plus grands). Ainsi, avec ce système il n'est pas possible de faire la différence entre des agrégats de billes magnétiques et des objets biologiques labélisés puisqu'il n'est pas possible de déterminer indépendamment le moment magnétique et la hauteur de passage à partir d'un seul champ dipolaire. La présence des agrégats de billes magnétiques provient du fait qu'il est nécessaire de mettre un excès de billes magnétiques fonctionnalisées par les anticorps spécifiques dans la matrice liquide pour augmenter les chances de labéliser correctement les objets biologiques.

Le document WO 2019/238857 A1 divulgue une méthode de détection magnétique au moyen d'une biopuce comprenant plusieurs paires de capteurs magnétorésistifs disposés de part et d'autre du canal microfluidique, notamment au-dessus et au-dessous du canal microfluidique. Les capteurs magnétorésistifs constituant une paire sont parfaitement alignés de part et d'autre du canal. Un objet biologique marqué passant entre les deux capteurs d'une même paire sera donc détecté de manière simultanée par ces deux capteurs. La paire des deux signaux émis simultanément par deux capteurs de la même paire (par exemple sur le canal, dit « TOP » et sous le canal, dit « BOTTOM ») est dite « synchronisée ».

Le procédé divulgué propose même de déterminer la trajectoire suivie par l'objet à partir du rapport des amplitudes des signaux mesurés par des paires de capteurs successifs disposés tout le long du canal microfluidique.

Il existe donc un besoin de déterminer la hauteur et le moment magnétique d'un objet biologique marqué à partir du rapport des amplitudes des signaux.

## RÉSUMÉ DE L'INVENTION

Dans ce contexte, l'invention concerne un procédé de détermination du moment magnétique d'un objet magnétique au moyen d'une biopuce selon la revendication indépendante 1.

Par signaux électriques synchronisés, on entend que les signaux électriques correspondent à la mesure du même objet magnétique, même si les capteurs ne sont pas alignés (les signaux électriques peuvent alors montrer un décalage temporel entre eux, par exemple proportionnel à la vitesse de déplacement de l'objet magnétique dans le canal microfluidique).

Grâce à l'analyse des signaux simultanés (synchronisés) et la détermination de la hauteur de passage, il est possible de déterminer le moment magnétique de l'objet détecté qui est, soit un objet biologique marqué par des billes magnétiques, soit un agrégat de billes magnétiques, soit des magnétosomes.

Le calcul de la hauteur de passage, moyennant l'utilisation d'une courbe de référence calibrée à partir d'un objet magnétique de calibration permet de déterminer de manière fiable la hauteur de passage de l'objet magnétique et donc le calcul de son moment magnétique. Dès lors, la discrimination des objets magnétiques à détecter est améliorée et permet de discriminer les objets biologiques marqués par un grand nombre de billes magnétiques par rapport à des agrégats constitués uniquement majoritairement de billes magnétiques (et ne comprenant pas d'objet magnétique). On peut ainsi discriminer les faux positifs liés aux agrégats des vrais positifs liés aux objets biologiques marqués.

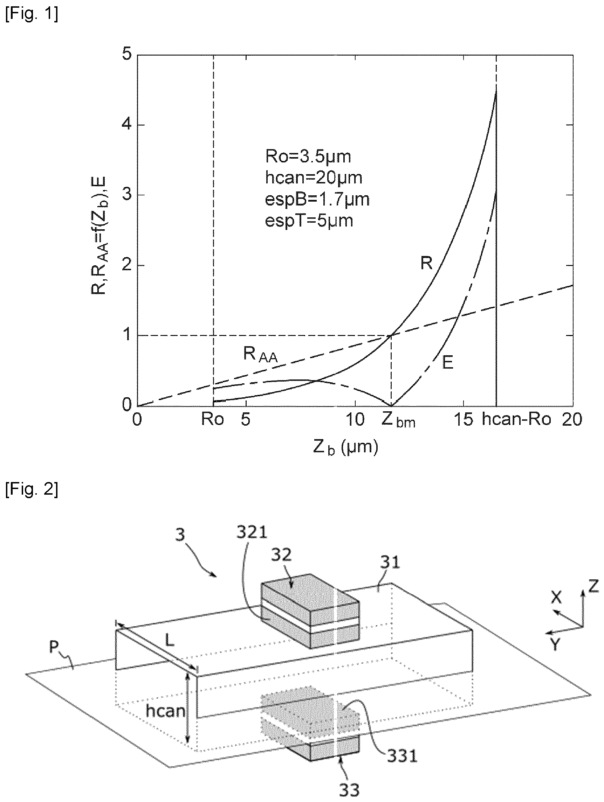

La [Fig. 1] présente un exemple de courbe de référence reliant le rapport R des amplitudes de signaux électriques de calibration en fonction de la hauteur de passage $z_b$ d'un objet magnétique de calibration de rayon Ro, dans le canal microfluidique de hauteur hcan. La hauteur de passage $z_b$ ne peut donc varier qu'entre Ro et hcan - Ro. Le rapport est représenté par la courbe continue et noté « R ». Ledit rapport R prend une valeur minimale lorsque la hauteur de passage $z_b$ de l'objet de calibration est minimale (lorsque $z_b$ = Ro). Selon l'invention, le rapport R augmente de manière non-linéaire à mesure que la hauteur de passage $z_b$ de l'objet magnétique de calibration augmente jusqu'à atteindre une valeur maximale (lorsque $z_b$ = hcan - Ro). La [Fig. 1] montre également un rapport $R_{AA}$, en trait discontinu, calculé selon l'art antérieur et notamment selon les enseignements du document WO 2019/238857 A, le calcul étant adapté aux présentes valeurs des paramètres. Le rapport $R_{AA}$ est linéaire avec la hauteur de passage $z_b$ et passe par 1 lorsque l'objet magnétique considéré est équidistant des capteurs de champ magnétique. On observe donc que les deux rapports R et $R_{AA}$ ne sont égaux que pour deux points ; $z_b$ = 0 et $z_b$ = $z_{bm}$. La figure montre également un écart en valeur absolue E (courbe en points-tirets) entre les deux rapports R et $R_{AA}$, qui peut être assimilé à une erreur absolue. L'écart E varie en fonction de la hauteur de passage $z_b$ et peut prendre des valeurs conséquentes dans certains cas (notamment autour de $z_{bm}/2$ ou lorsque $z_b$ est supérieur à $z_{bm}$, où R tend vers l'infini).

La courbe de référence permet donc d'améliorer l'estimation de la hauteur de passage de l'objet magnétique et améliorer ainsi la détermination de son moment magnétique.

Il convient d'ajouter que la détermination préliminaire de la hauteur de passage ne sert pas uniquement à réduire une erreur systématique. Elle permet en effet, une fois la hauteur de passage déterminée, de déterminer un signal théorique d'une bille dont on connait le moment magnétique par des mesures préliminaires avec un magnétomètre. Le rapport du signal expérimental sur le signal théorique d'une bille nous permet de déterminer le nombre de billes N contenues par l'objet détecté (à l'origine du signal expérimental) et par conséquent le moment magnétique qui est alors N fois le moment magnétique d'une bille.

Le procédé est également compatible avec une biopuce de l'art antérieur (telle que celle divulguée par le document WO 2019-238857 A).

Le procédé permet également de caractériser des agrégats de billes magnétiques (ne comprenant pas d'objet biologique) développés pour différentes applications. En effet, les techniques de mesure d'aimantation macroscopiques, tel que les magnétomètres à échantillons vibrants ou un magnétomètre à « SQUID » ne permettent pas la mesure du moment magnétique d'échantillons micrométriques uniques.

Par disposés de part et d'autre du canal microfluidique, on entend disposés de part et d'autre de la hauteur du canal microfluidique.

Avantageusement, les capteurs de champ magnétique sont disposés en vis-à-vis l'un de l'autre et alignés selon la direction normale.

Avantageusement, la courbe de référence est déterminée à partir de l'objet magnétique de calibration présentant un moment magnétique de calibration indépendant du moment magnétique de l'objet magnétique. Ainsi, la courbe de référence peut être établie quel que soit les objets magnétiques finaux à caractériser.

Avantageusement, les signaux électriques issus des capteurs de champ magnétique sont proportionnels aux champs dipolaires émis par l'objet magnétique et perçus par les capteurs de champ magnétique. Autrement dit, le rapport des amplitudes des signaux électriques de caractérisation est égal au rapport des amplitudes du champ dipolaire émis par l'objet magnétique de calibration sur chacun des capteurs de champ magnétique.

Avantageusement, le signal électrique issu de chaque capteur de champ magnétique est égal au champ dipolaire émis par l'objet magnétique multiplié par un facteur de sensibilité du capteur de champ magnétique, le facteur de sensibilité du capteur de champ magnétique étant avantageusement linéaire et préférentiellement égal à 2 %/mT, voire à 1 %/mT. Avantageusement, les facteurs de sensibilité des deux capteurs de champ magnétique sont identiques.

De manière encore plus avantageusement, chaque signal électrique de calibration est égal au champ dipolaire émis par l'objet magnétique de calibration au niveau d'un capteur de champ magnétique de calibration multiplié par un facteur de sensibilité de calibration, les facteurs de sensibilité de calibration étant égaux aux facteurs de sensibilité des deux capteurs de champ magnétique.

Avantageusement, le procédé comprend une étape de détermination de la courbe de référence comprenant les sous-étapes suivantes :

- pour différentes hauteurs de passage de l'objet magnétique de calibration entre deux capteurs de champ magnétique de calibration :

    - déterminer le champ magnétique dipolaire émis par l'objet magnétique de calibration et perçu par l'un des capteurs de champ magnétique de calibration ;

    - déterminer le champ magnétique dipolaire émis par l'objet magnétique de calibration et perçu par l'autre des capteurs de champ magnétique de calibration ;

- calculer le rapport des amplitudes des champs magnétiques dipolaires perçus par les capteurs de champ magnétique de calibration en fonction des différentes hauteur de passage de l'objet magnétique de calibration.

Avantageusement, l'étape de réception comprend également une sous-étape d'identification des signaux électriques synchronisés comprenant la mesure d'un écart temporel entre les signatures caractéristiques des deux signaux électriques, la synchronisation étant identifiée lorsque l'écart temporel est compris dans une gamme temporelle prédéterminée, la gamme temporelle prédéterminée étant préférentiellement déterminée à partir d'une vitesse de déplacement de l'objet magnétique. Chaque signature caractéristique correspondant à la mesure de l'objet magnétique par l'un des capteurs de champ magnétique.

Avantageusement, les amplitudes des signaux électriques sont préférentiellement estimées au niveau des signatures caractéristiques de chaque signal électrique.

Avantageusement, l'étape de réception comprend une sous-étape d'identification des signatures caractéristiques dans chacun des signaux électriques à partir de critères de forme des signaux électriques.

Avantageusement, l'objet magnétique est un objet biologique marqué au moyen de billes magnétiques.

Avantageusement, le procédé comprend également une étape de calcul du nombre de billes magnétiques associé à l'objet biologique marqué à partir du moment magnétique calculé et du moment magnétique d'une seule bille magnétique. Le moment magnétique d'une seule bille magnétique peut être déduit d'une mesure de moment magnétique d'une assemblée de billes magnétiques, la mesure de moment magnétique étant par exemple réalisée au moyen d'un magnétomètre à échantillons vibrants.

Avantageusement, les capteurs de champ magnétique sont des capteurs magnétorésistifs et préférentiellement basés sur l'effet de magnétorésistance géante (dite « GMR » pour « Giant MagnetoResistance » en anglais) ou sur l'effet de magnétorésistance tunnel (dite « TMR » pour « Tunnel MagnetoResistance » en anglais).

L'invention concerne également un dispositif de détermination du moment magnétique d'un objet magnétique selon la revendication indépendante 12.

L'invention concerne aussi un programme d'ordinateur selon la revendication indépendante 14 comprenant des instructions qui conduisent le dispositif précité à exécuter les étapes du procédé de détermination du moment magnétique d'un objet magnétique selon l'invention.

Un support lisible par ordinateur, sur lequel est enregistré le programme d'ordinateur précité et ne faisant pas partie de l'invention, est également divulgué.

L'invention concerne aussi un procédé de comptage d'objets biologiques selon la revendication dépendante 10.

Le procédé de comptage permet ainsi de compter les objets biologiques marqués. Il permet par exemple d'obtenir un histogramme des hauteurs de passage des différents objets magnétiques détectés. Il permet également d'obtenir un histogramme des moments magnétiques (et donc du nombre de billes magnétiques) associés aux objets magnétiques détectés. Le procédé de comptage peut également permettre un comptage des objets biologiques simultanément à la circulation de la matrice liquide comprenant les objets biologiques marqués dans le canal microfluidique de la biopuce.

Avantageusement, le procédé de comptage comprend l'étape de détermination du moment magnétique seuil, ladite étape comprenant les sous-étapes suivantes :

- faire circuler la matrice liquide sans objet biologique, dite « contrôle négatif », dans le canal microfluidique de la biopuce et déterminer le moment magnétique d'objets magnétiques passant entre les capteurs de champ magnétique au moyen du procédé de détermination du moment magnétique selon l'invention ; et
- fixer le moment magnétique seuil à partir d'une répartition statistique des moments magnétiques déterminés.

L'invention concerne aussi un système de comptage d'objets biologiques selon la revendication dépendante 13.

L'invention concerne également un programme d'ordinateur selon la revendication indépendante 15 comprenant des instructions qui conduisent le dispositif de comptage selon l'invention à exécuter les étapes du procédé de comptage de l'invention.

Un support lisible par ordinateur, sur lequel est enregistré le programme d'ordinateur précité et ne faisant pas partie de l'invention, est également divulgué.

L'invention et ses différentes applications seront mieux comprises à la lecture de la description qui suit et à l'examen des figures qui l'accompagnent.

## BRÈVE DESCRIPTION DES FIGURES

Les figures sont présentées à titre indicatif et nullement limitatif de l'invention. Sauf précision contraire, un même élément apparaissant sur des figures différentes présente une référence unique.

[Fig. 1] présente un exemple de courbe de référence mis en œuvre par le procédé de détermination selon l'invention.

[Fig. 2] représente schématiquement une biopuce qui peut être mise en œuvre par le procédé de détermination selon l'invention.

[Fig. 3] représente schématiquement un exemple d'empilement de couches permettant d'obtenir un capteur de champ magnétique tel qu'utilisé dans la biopuce de la [Fig. 2].

[Fig. 4] montre un exemple de l'application d'un champ magnétique sur un empilement de couches tel qu'illustré à la [Fig. 3].

[Fig. 5] représente, en vue de dessus, un exemple de géométrie d'un capteur de champ magnétique tel qu'utilisé dans la biopuce de la [Fig. 2].

[Fig. 6] représente un exemple de disposition de capteurs de champ magnétique au sein d'une biopuce telle qu'illustrée dans la [Fig. 2].

[Fig. 7] représente un premier exemple de signaux électriques issus d'une biopuce telle qu'illustrée dans la [Fig. 2] lors du passage d'un objet magnétique entre ses deux capteurs de champ magnétique.

[Fig. 8] représente un deuxième exemple de signaux électriques issus d'une biopuce telle qu'illustrée dans la [Fig. 2] lors du passage d'un objet magnétique entre ses deux capteurs de champ magnétique.

[Fig. 9] représente un troisième exemple de signaux électriques issus d'une biopuce telle qu'illustrée dans la [Fig. 2] lors du passage d'un objet magnétique entre ses deux capteurs de champ magnétique

[Fig. 10] représente schématiquement un mode de mise en œuvre du procédé de détermination selon l'invention.

[Fig. 11] représente un mode de mise en œuvre d'une étape du procédé de la [Fig. 10].

[Fig. 12] représente un exemple de champ dipolaire calculé à différentes positions et pour différents nombres de billes magnétiques.

[Fig. 13] représente schématiquement un mode de réalisation d'un dispositif de détermination selon l'invention.

[Fig. 14] représente un exemple de chaîne d'acquisition du dispositif de la [Fig. 13].

[Fig. 15] illustre schématiquement un mode de mise en œuvre d'un procédé de comptage selon l'invention.

[Fig. 16] représente schématiquement un principe de marquage d'un objet biologique afin de pouvoir être compté par le procédé de la [Fig. 15].

[Fig. 17] représente un exemple d'objet biologique 5 marqué.

[Fig. 18] représente un exemple de résultat obtenu au moyen du procédé de comptage de la [Fig. 15].

## DESCRIPTION DÉTAILLÉE

La [Fig. 2] représente schématiquement une biopuce 3 qui peut être mise en œuvre par un procédé 1 selon l'invention. Le document antérieur WO 2019-238857 A décrit un autre mode de réalisation d'une biopuce 3 pouvant être mise en œuvre par le procédé 1. La biopuce 3, dans l'exemple de la [Fig. 2], comprend un canal microfluidique 31 qui s'étend dans un plan P. La figure montre une portion de ce canal microfluidique 31 qui s'étend d'ailleurs selon une direction Y, parallèle au plan P. Le canal possède avantageusement une entrée et une sortie de manière à pouvoir faire circuler un fluide à analyser, par exemple selon la direction Y, voire dans le sens des Y croissants. Le canal microfluidique 31 peut présenter une section rectangulaire, telle qu'illustrée ici, ou une section différente, dès lors qu'elle permet la disposition de capteurs de champ magnétique. La section du canal microfluidique 3 présente par exemple une hauteur $h_{can}$, mesurée selon une direction Z (dite « direction normale ») perpendiculaire au plan P, comprise entre 20 $\mu$m et 25 $\mu$m. Elle peut présenter une largeur L, mesurée selon une direction X perpendiculaire aux directions Y et Z, comprise entre 100 $\mu$m et 150 $\mu$m.

La biopuce 3 comprend également des premier et deuxième capteurs de champ magnétique 31, 32. Les capteurs 32, 33 sont disposés de part et d'autre du canal microfluidique 31. Le premier capteur 32, dit également capteur « haut » ou « supérieur », voire « top » en anglais, est par exemple disposé sur le canal 31. Un deuxième capteur 33, dit également capteur « bas » ou « inférieur » ou « bottom » en anglais, est par exemple disposé sous le canal 31. Dans le mode de réalisation de la [Fig. 2], les capteurs sont disposés en vis-à-vis l'un de l'autre. Ils sont notamment alignés, l'un au-dessus de l'autre selon la direction normale Z. Par « en vis-à-vis », on entend que les capteurs top et bottom 31, 32 présentent chacun une orientation et qu'ils sont orientés de manière opposés l'un par rapport à l'autre. En d'autres mots, chaque capteur 31, 32 peut comprendre une partie sensible 321, 331, permettant de réaliser la mesure d'un champ magnétique. Ces deux parties sensibles 321, 331 peuvent alors être orientées en regard l'une de l'autre. Les parties sensibles 321, 331 sont d'ailleurs avantageusement orientées vers le canal microfluidique 3, voire au contact de celui-ci.

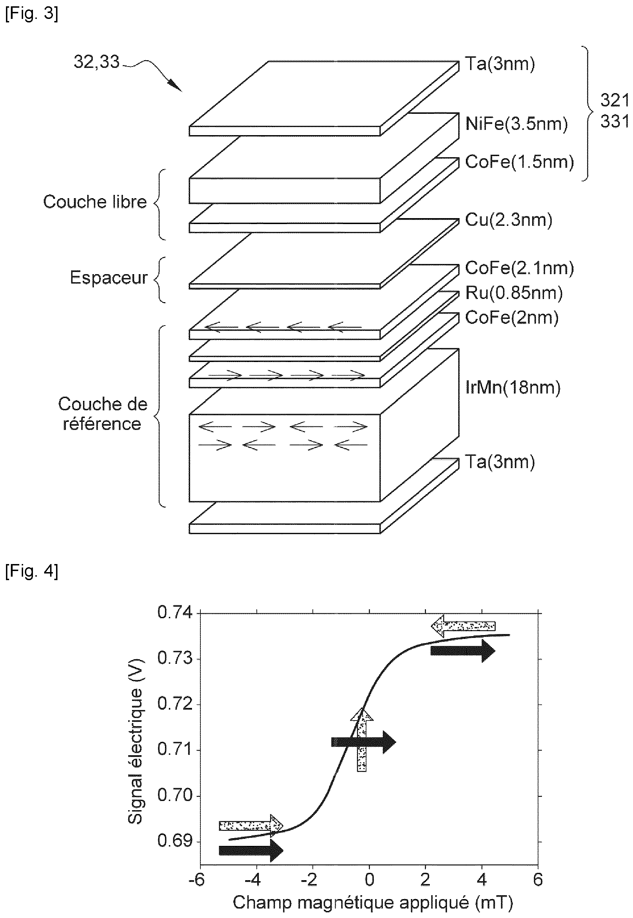

La [Fig. 3] montre un exemple d'empilement de couches permettant d'obtenir un capteur de champ magnétique 32, 33. Il s'agit ici d'un capteur magnétorésistif. Il pourrait également s'agit de capteur dit « SQUID » mettant en œuvre une boucle supraconductrice. Les couches choisies dans cet exemple permettent de manière d'obtenir un effet de magnétorésistance géante (dite « GMR » pour « Giant MagnetoResistance » en anglais). Elles pourraient également être choisies pour obtenir un effet de de magnétorésistance tunnel (dite « TMR » pour « Tunnel MagnetoResistance » en anglais). Dans les deux cas (et surtout dans une utilisation de capteur) la résistance de l'empilement varie en fonction de l'orientation de l'aimantation d'une couche dite « libre », par rapport à l'orientation de l'aimantation d'une couche dite « de référence ».

La [Fig. 4] montre par exemple l'effet de l'application d'un champ magnétique sur un empilement de couches tel qu'illustré à la [Fig. 3]. Le champ magnétique est appliqué antiparallèle à l'orientation de l'aimantation de la couche de référence (l'aimantation de la couche de référence est considérée comme fixe). Lorsque les deux aimantations, des couches libre et de référence, sont parallèles, alors la résistance de l'empilement est faible. La tension mesurable à ses bornes, pour un courant de polarisation donné, est donc faible. À l'inverse, lorsque les deux aimantations, des couches libres et de référence, sont antiparallèles, alors la résistance de l'empilement est élevée. La tension mesurable à ses bornes est donc élevée.

Il existe une plage dans laquelle les aimantations des couches libres et de référence ne sont ni parallèles ni antiparallèles, mais où elles forment un angle aigu. Dans cette plage, la résistivité varie linéairement avec l'angle entre les deux aimantations. La pente, par exemple en %/mT (en considérant le taux de variation de la résistance) ou en $\Omega$/mT ou encore en V/mT (lorsque l'on considère la tension aux bornes de l'empilement pour un courant prédéterminé), dans cette plage correspond donc à la sensibilité de l'empilement. C'est cette plage qui est préférentiellement considérée pour réaliser les capteurs top et bottom 32, 33 puisqu'elle permet de mesurer les petites variations d'orientation de l'aimantation de la couche libre, par exemple, lorsque cette dernière est perturbée par un objet magnétique tel qu'un objet biologique marqué.

La couche libre forme avantageusement la partie sensible 321, 331 des capteurs 32, 33.

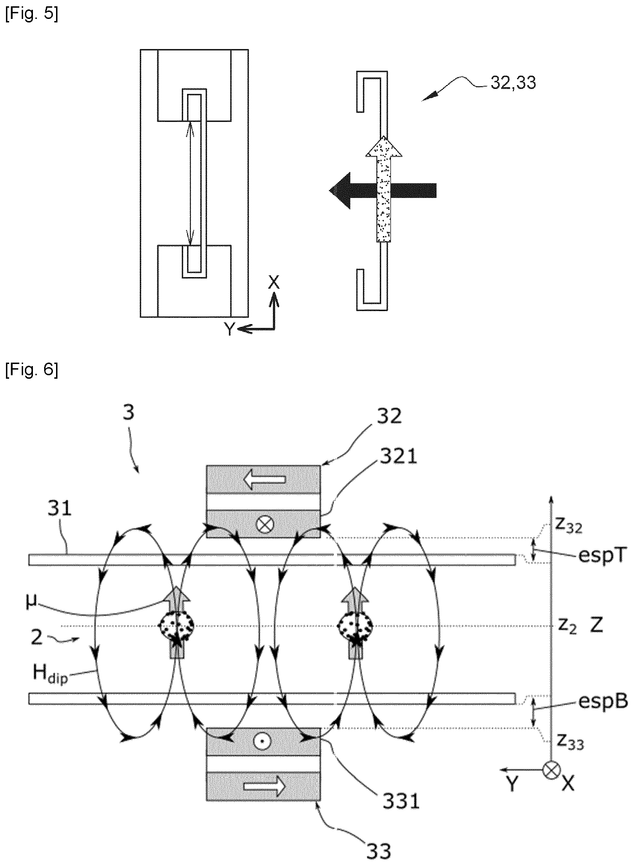

Les [Fig. 3] et [Fig. 5] montrent un exemple d'empilement de couches et de géométrie de l'empilement permettant d'obtenir des capteurs magnétorésistifs 32, 33 capables de fonctionner dans la plage linéaire telle que décrite. L'empilement présenté par la [Fig. 3] comprend deux couches ferromagnétiques espacées l'une de l'autre par un matériau non-magnétique, dit « espaceur ». Dans le cas d'un empilement mettant en œuvre l'effet GMR, tel que présenté dans la [Fig. 3], l'espaceur est une couche d'un métal non-magnétique tel que le cuivre. Dans le cas d'un empilement mettant en œuvre l'effet TMR, l'espaceur est une couche diélectrique, par exemple en MgO. La couche ferromagnétique dite « de référence », présente, sur une de ses faces, une aimantation nette fixe (c'est-à-dire que son orientation et son amplitude sont constante). La couche de référence comprend par exemple deux sous-couches ferromagnétiques, couplées de manière antiferromagnétiques (par exemple par couplage d'échange au moyen de la sous-couche de Ru de 0,85 nm d'épaisseur) de manière à former une sous-couche en apparence antiferromagnétique, dite « antiferromagnétique synthétique » ou « SyAF ». La sous-couche SyAF présente alors une sous-couche supérieure présentant une aimantation nette fixe. La couche de référence peut également comprendre une sous-couche antiferromagnétique (naturelle, par opposition à synthétique) en IrMn, permettant de renforcer l'aimantation de la sous-couche SyAF.

La couche ferromagnétique, dite « libre », présente une aimantation libre. Pour cela, cette couche peut être réalisée à partir d'un matériau magnétique doux tel que CoFe ou NiFe, ou à partir d'un empilement de sous-couches du type CoFe/NiFe.

L'empilement est avantageusement disposé entre deux électrodes métalliques, par exemple en Ta, permettant de faire circuler un courant électrique en son sein.

Les aimantations des couches libre et de référence sont préférentiellement parallèles au plan des couches. L'orientation de l'aimantation de la couche de référence est imposée, par exemple par la couche d'IrMn. L'orientation en champ nul de l'aimantation de la couche libre peut être imposée par un autre moyen, par exemple au moyen d'une anisotropie de forme. Pour cela, la couche libre présente avantageusement une forme allongée selon une direction dans le plan des couches. De la sorte, l'aimantation de la couche libre va s'orienter spontanément selon cette direction. Dans le mode de réalisation de la [Fig. 5], la couche libre présente une forme de joug (dit aussi forme de « C » ou de « fer à cheval ») avec un tronçon long et étroit, localisé au niveau de l'empilement. Cette forme en joug permet d'avoir une aimantation mono-domaine de la couche libre. Le tronçon long et étroit crée également une anisotropie de forme qui va orienter l'aimantation de la couche libre selon la grande longueur (en l'occurrence selon l'axe X). L'orientation de la grande longueur du tronçon est avantageusement choisie perpendiculaire à l'orientation de l'aimantation de la couche de référence (cette dernière étant, en l'occurrence, orientée selon l'axe Y). De la sorte, les aimantations des couches libre et de référence sont, en l'absence de perturbation, perpendiculaires l'une par rapport à l'autre. De cette manière, les capteurs magnétorésistifs 32, 33 fonctionnent dans la plage linéaire, là où la sensibilité est maximale et constante.

La [Fig. 6] montre un exemple de disposition des capteurs de champ magnétique 32, 33, en l'occurrence magnétorésistifs, de part et d'autre du canal microfluidique 31. Dans cet exemple, les capteurs 32, 33 sont alignés selon la direction Z. Les parties sensibles 321, 331, comprenant ici les couches libres des capteurs 32, 33, sont orientées en regard du canal 31. Elles sont également avantageusement disposées à proximité du canal microfluidique 3. Les parties sensibles 321, 331 peuvent être au contact du canal microfluidique 31. Toutefois, ce cas de figure peut être difficile à réaliser de manière pratique car des limites technologiques, par exemple relatives aux étapes de fabrication de la biopuce 3, peuvent imposer une séparation entre les capteurs 32, 33 et le canal 31. Le capteur top 32 est par exemple séparé du canal 31 d'une distance espT du canal 31, par exemple comprise entre 3 $\mu$m et 7 $\mu$m (dans l'exemple de la [Fig. 1] la valeur de 5 $\mu$m est considérée). Cette distance correspond par exemple à une couche de matériau séparant le canal 31 du capteur top 32 et nécessaire à la réalisation du canal 31 et/ou du capteur top 32. De la même manière, le capteur bottom 33 est par exemple séparé d'une distance espB du canal 31, par exemple compris entre 0,3 $\mu$m et 3 $\mu$m, par exemple égale à 1,7 $\mu$m.

Par hauteur, on entend une distance mesurée selon la direction normale Z. Toutefois, cette nomenclature n'est pas à prendre au mot car les effets techniques sont invariables par rotation autour de la direction Y. En d'autres termes, les capteurs 32, 33 pourraient être disposés sur les côtés du canal 31 plutôt que sur le dessus et le dessous du canal 31. La hauteur devient donc une largeur. Toutefois, afin de simplifier la description, seuls des exemples prenant en compte une hauteur sont considérés par la suite.

Dans la [Fig. 6], les aimantations des couches libres des capteurs 32, 33 sont antiparallèles entre elles et orientées selon la direction X (notamment transverse à la direction Y de l'écoulement). Les aimantations des couches de référence des capteurs 32, 33 sont également antiparallèles entre elles et orientées parallèlement à la direction Y de l'écoulement.

La [Fig. 6] montre un objet magnétique 2 à deux positions différentes dans le canal microfluidique 3. L'objet magnétique 2 suit par exemple l'écoulement d'une matrice fluide dans le canal 3, l'écoulement étant par exemple dans le sens des Y croissants. L'objet est donc d'abord à droite et ensuite à gauche, un bref instant après. L'objet magnétique 2 suit l'écoulement à une hauteur constante z, mesurée selon la direction normale Z.

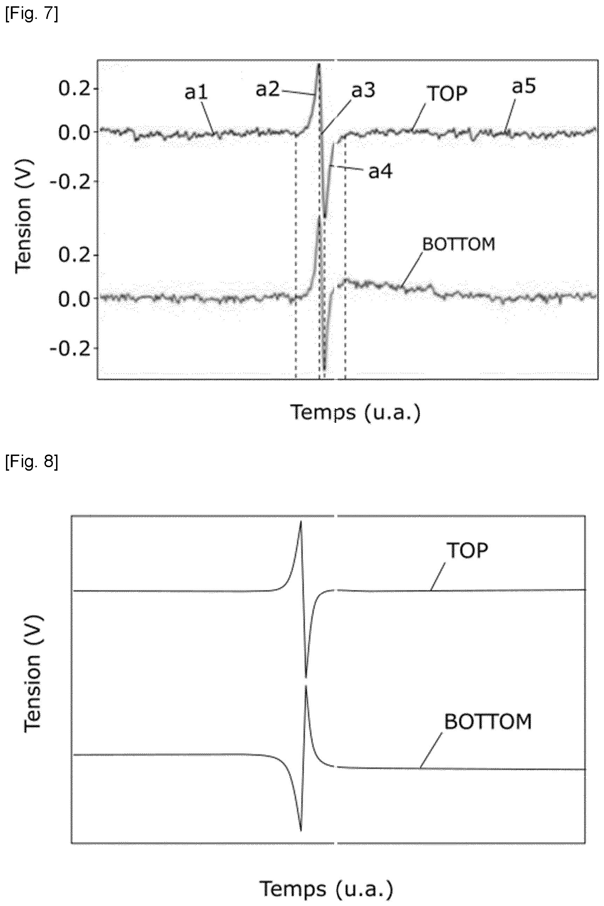

L'objet magnétique 2 est par exemple un objet biologique marqué (tel qu'une molécule ou une cellule), un magnétosome (une molécule présentant un moment magnétique intrinsèque) ou encore un agrégat de billes magnétiques. Par billes magnétiques, dites « magnetic beads » en anglais, on entend des microparticules ou nanoparticules magnétiques destinées à être attachée à un objet biologique. Dans la [Fig. 6], on considère un objet biologique marqué. L'objet magnétique 2 porte un moment dipolaire $\mu$. La [Fig. 6] illustre l'orientation du moment dipolaire $\mu$, orienté sensiblement selon la direction Z. Le moment dipolaire $\mu$ de l'objet magnétique 2 est dû à une fonctionnalisation d'un objet biologique avec des billes magnétiques (illustrées par les petits points noirs sur le cercle blanc). On parle alors d'un objet biologique marqué (principe de marquage illustré en [Fig. 8]).

La composante selon l'axe Y du champ magnétique $H_{dip}$ émis par l'objet magnétique 2 est perçu par les capteurs top 32 et bottom 33. La [Fig. 6] montre deux lignes de champ $H_{dip}$ issues de l'objet magnétique 2 ainsi que la direction du champ $H_{dip}$. Selon que l'objet magnétique 2 est à droite ou à gauche des capteurs 32, 33 (c'est-à-dire en amont ou en aval des capteurs), le lobe des lignes de champ couplé aux capteurs 32, 33 n'est pas le même. Le champ magnétique perçu n'est pas non plus orienté dans le même sens pour les deux capteurs 32, 33.

Lorsque l'objet magnétique 2 est :

- à droite (sur la figure) des capteurs 32, 33, c'est-à-dire en amont (suivant l'écoulement selon Y), alors :

    - le lobe du champ magnétique rayonné (celui de gauche en l'occurrence) tend à orienter l'aimantation de la couche libre du capteur top 32 à gauche ; et

    - l'aimantation de la couche libre du capteur bottom 33 à droite ;

- à gauche (sur la figure) des capteurs 32, 33, c'est-à-dire en aval (suivant l'écoulement selon Y), alors :

    - le lobe du champ magnétique rayonné (celui de droite en l'occurrence) tend à orienter l'aimantation de la couche libre du capteur top 32 à droite ; et

    - l'aimantation de la couche libre du capteur bottom 33 à gauche.

L'effet du changement de lobe couplé aux couches libres de capteurs 32, 33 lors du passage de l'objet magnétique 2 est perçu (et donc mesurable).

La [Fig. 7] illustre un exemple de signaux électriques issus des deux capteurs 32, 33 en fonction du temps, lors du passage d'un objet magnétique 2 entre les deux capteurs 32, 33. La courbe TOP représente par exemple le signal électrique émis par le capteur top 32 et la courbe BOTTOM représente par exemple le signal émis par le capteur bottom 33. Chaque signal montre une signature caractéristique de la mesure d'un objet magnétique 2. Il s'agit en l'occurrence d'un premier pic de tension immédiatement suivi d'un second pic dont la polarité est inversée.

La forme particulière de cette signature s'explique comme suit. Au point a1, la courbe TOP présente une tension nulle, signe qu'aucun objet magnétique n'est mesuré. L'objet magnétique 2 est en amont des capteurs 32, 33 et son champ rayonné est encore indétectable. Au point a2, un signal électrique non nul montre que l'aimantation de la couche libre du capteur top 32 est perturbée par un objet magnétique 2. Un premier lobe du champ rayonné de l'objet magnétique influence donc l'aimantation de la couche libre. L'objet est donc entre les capteurs 32, 33 et légèrement en amont des capteurs 32, 33. Au point a4, le signal électrique non nul montre que l'aimantation de la couche libre du capteur top 32 est également perturbée par un objet magnétique 2. En revanche le signal est négatif, signe qu'un deuxième lobe du champ rayonné de l'objet magnétique influence l'aimantation de la couche libre selon une direction opposée. L'objet est donc entre les capteurs 32, 33 et légèrement en aval des capteurs 32, 33. Au point a3, le signal est nul, signe que l'aimantation de la couche libre a repris sa direction d'équilibre. L'objet magnétique est disposé entre les deux capteurs 32, 33 et est exactement centré (selon la direction d'écoulement) par rapport aux capteurs 32, 33. Les deux lobes du champ rayonné de l'objet magnétique influencent l'aimantation de la couche à parts égales. Au point a5, l'objet magnétique est en aval des capteurs 32, 33 mais est disposé trop loin des capteurs pour que son champ soit ressenti.

L'étendue d'une signature caractéristique dépend uniquement de la vitesse de passage de l'objet au niveau des capteurs. Elle dépend donc principalement de la vitesse d'écoulement dans le canal microfluidique 31.

Dans cet exemple, les courbes TOP et BOTTOM présentent des signatures caractéristiques pratiquement identiques. Lorsque la courbe TOP est positive, la courbe BOTTOM l'est également. Les maximas et minimas des deux courbes TOP, BOTTOM apparaissent aux mêmes instants et atteignent des valeurs comparables. Ceci indique que l'objet est détecté de manière simultanée par les deux capteurs (rappelons que dans cet exemple, les capteurs sont alignés).

Lorsque les capteurs 32, 33 sont alignés selon une direction transverse à l'écoulement, en l'occurrence selon la direction Z, le passage de l'objet magnétique 2 entre les deux capteurs 32, 33 déclenche une réponse simultanée et de forme similaire mais dont l'amplitude peut dépendre de la hauteur $z_2$ de passage de l'objet 2.

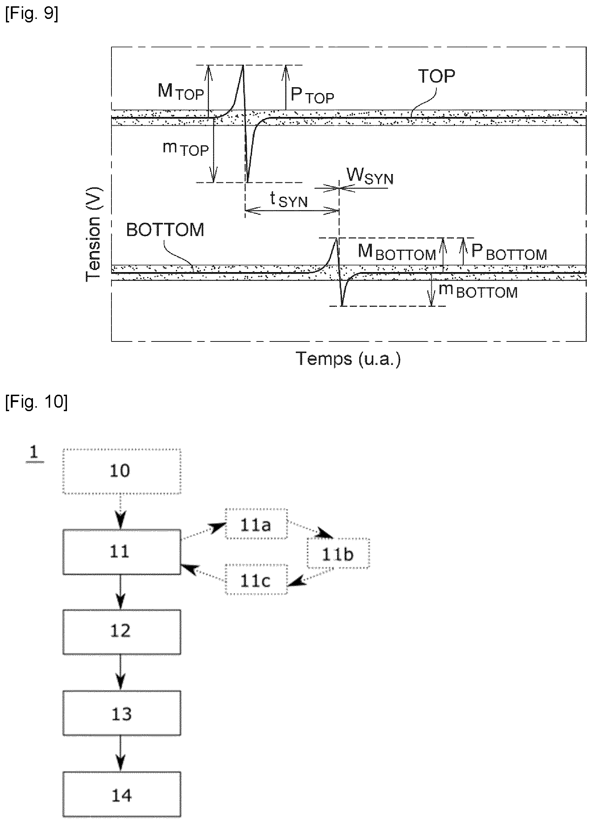

La réponse à un même objet magnétique peut toutefois varier de deux façons différentes, illustrées par les [Fig. 8] et [Fig. 9].

Par exemple, dans la [Fig. 8], la polarité de la signature caractéristique de la courbe BOTTOM est inversée par rapport à celle de la courbe TOP. En revanche les signatures caractéristiques sont simultanées. Ceci vient par exemple du fait que l'orientation de l'aimantation de la couche libre du deuxième capteur 33 est inversée par rapport à l'orientation de l'aimantation de la couche de référence du deuxième capteur 33.

Dans la [Fig. 9], la courbe BOTTOM présente une amplitude maximale plus faible que l'amplitude maximale de la courbe TOP. Les polarités des signatures sont en revanche identiques. L'objet magnétique 2 est donc passé plus près du capteur top 32 que du capteur bottom 33. Les signatures caractéristiques des deux courbes n'interviennent pas aux mêmes instants. La signature de la courbe TOP intervient avant celle de la courbe BOTTOM. Ceci peut venir du fait que les capteurs de champ 32, 33 ne sont pas alignés selon la direction Z (contrairement à ce qui est montré par la [Fig. 6]. Le capteur top 32 (dont est issue la courbe TOP) est placé en amont du capteur bottom 33 (dont est issue la courbe BOTTOM) par rapport à la direction de l'écoulement dans le canal microfluidique 31. Dès lors, l'objet magnétique 2 perturbe d'abord le capteur top 32 et ensuite le capteur bottom 33. Le décalage temporel $t_{SYN}$ entre ces deux signatures (mesuré au centre des signatures, lorsque le signal est nul) dépend de la vitesse de passage de l'objet magnétique 2 au niveau des capteurs 32, 33. Le décalage $t_{SYN}$ dépend donc de la vitesse d'écoulement dans le canal microfluidique 31.

La [Fig. 6] montre, selon la direction normale Z, la hauteur de passage $z_2$ de l'objet magnétique 2 dans le canal microfluidique 3. Cette hauteur de passage $z_2$ est à prendre en considération par rapport aux hauteurs respectives $z_{32}$, $z_{33}$ des capteurs top 32 et bottom 33. Les hauteurs $z_{32}$, $z_{33}$ prises en compte sont d'ailleurs préférentiellement les hauteurs, selon la direction normale Z, des couches libres de chaque capteur.

Lorsque la hauteur de passage $z_2$ est équidistante des hauteurs $z_{32}$ (du capteur top) et $z_{33}$ (du capteur bottom), les signaux mesurés présentent les mêmes amplitudes (telles qu'illustrées par les [Fig. 7] et [Fig. 8]). Lorsque la hauteur de passage $z_2$ de l'objet magnétique 2 n'est pas équidistante, c'est-à-dire que l'objet magnétique 2 passe plus près du capteur top 32 ou du capteur bottom 33, les signaux mesurés présentent alors des amplitudes différentes (telles qu'illustrées par la [Fig. 9]).

Le procédé 1 selon l'invention permet de détecter le passage d'un objet magnétique 2 entre les deux capteurs 32, 33 en recourant à la détermination de son moment magnétique $\mu$. Pour cela, le procédé 1 propose de déterminer, avec précision, la hauteur de passage $z_2$ de l'objet magnétique entre les deux capteurs 32, 33. La raison en est que le champ rayonné au niveau du capteur top 32 est proportionnel à $\mu/(z_2 - z_{32})^3$. Une bonne estimation de la hauteur de passage permet donc d'estimer le moment magnétique de l'objet magnétique 2.

Le procédé 1 peut ainsi être appliqué à la détection ou au comptage d'objets magnétiques ou encore au tri d'objets magnétiques en fonction de leur moment magnétique.

La [Fig. 10] représente schématiquement un mode de mise en œuvre du procédé 1. Selon une première étape, le procédé 1 prévoit la réception 11 des signaux électriques issus respectivement des capteurs top 32 et bottom 33. Il s'agit par exemple des signaux électriques illustrés par des courbes TOP et BOTTOM dans la [Fig. 9]. Ces signaux électriques correspondent au passage de l'objet magnétique 2 dans le canal microfluidique 31 à une hauteur de passage $z_2$ qui doit être déterminée. Les signaux reçus sont particuliers en ce qu'ils correspondent au passage du même objet 2. Ils sont d'ailleurs dits « synchronisés ». Ils présentent par exemple des signatures caractéristiques de la mesure d'un même objet magnétique 2 et l'écart entre ces signatures caractéristiques coïncide par exemple avec la vitesse d'écoulement dans le canal microfluidique 31.

Le procédé 1 comprend une étape de calcul 12 de la hauteur de passage $z_2$ de l'objet magnétique 2 à partir des signaux électriques synchronisés précédemment reçus. Pour cela, le procédé 1 prévoit l'utilisation d'une courbe de référence $R(z_b)$ pour obtenir la hauteur $z_2$ de l'objet magnétique 2 dans le canal 3 à partir du rapport des amplitudes des signaux électriques synchronisés.

Pour l'appliquer aux signaux synchronisés de la [Fig. 9], le rapport des amplitudes est par exemple obtenu à partir des amplitudes maximales positives $M_{TOP}$, $M_{BOTTOM}$ des signaux synchronisés TOP, BOTTOM, en calculant par exemple $M_{TOP}/M_{BOTTOM}$. Le rapport des amplitudes pourrait également être obtenu à partir des amplitudes maximales négatives $m_{TOP}$, $m_{BOTTOM}$ des signaux synchronisés TOP, BOTTOM, en calculant par exemple $m_{TOP}/m_{BOTTOM}$. Il est également envisageable de calculer une combinaison linéaire des deux rapports précités $a \times M_{TOP}/M_{BOTTOM} + b \times m_{TOP}/m_{BOTTOM}$.

Le rapport des amplitudes obtenues est comparé à la courbe de référence $R(Z_b)$, telle qu'illustrée par la [Fig. 1]. La [Fig. 1] illustre un exemple de courbe de référence $R(z_b)$. La courbe R relie la hauteur de passage $z_b$ d'un objet magnétique de calibration dans le canal microfluidique 31 en fonction d'un rapport R d'amplitudes de signaux électriques de calibration. Une lecture directe de la courbe fournit une hauteur de passage (notée $z_b$) de l'objet magnétique de calibration dans la [Fig. 1] qui est égale à la hauteur de passage $z_2$ de l'objet magnétique 2 ciblé (en d'autres termes, $z_2 = z_b$).

Le procédé 1 prévoit, selon une troisième étape, le calcul 13 du moment magnétique $\mu$ de l'objet magnétique 2. Pour cela, la hauteur de passage $z_2$ précédemment déterminée est utilisée. Elle est comparée au premier signal électrique et/ou au deuxième signal électrique. En effet, chaque signal électrique est proportionnel au champ magnétique champ dipolaire perçu au niveau des capteurs 32, 33 qui, elle-même, dépendent de la distance de l'objet magnétique 2 par rapport aux capteurs 32, 33 et de son moment magnétique $\mu$.

Par exemple, une manière de faire est de considérer le signal électrique $V_{32}$ mesuré par le capteur top 32. Il peut être approximé par

$$V_{23} \approx S_{32} \cdot \mu / r^3$$

où $S_{32}$ est la sensibilité du capteur top 32, $\mu$ est le moment magnétique de l'objet ciblé 2 et $r$ est la distance de l'objet ciblé 2 par rapport à r. Lorsque la tension mesurée aux bornes du premier capteur est maximale (par exemple $M_{TOP}$ sur la [Fig. 9]), l'objet magnétique 2 est positionné sous le capteur top 32. Il ne se trouve pas exactement à l'aplomb du capteur top 32 (puisque nous avons vu que le signal mesuré y est nul, comme discuté en référence à la [Fig. 7]) mais sa distance r vis-à-vis du premier capteur 32 est très proche de la hauteur de passage $z_2$. On peut donc estimer la tension $V_{32}$ par

$$V_{32} = S_{32} \cdot \mu \cdot z_2$$

On obtient donc le moment magnétique $\mu$ de l'objet magnétique 2 en calculant

$$\mu = \frac{V_{32}}{S_{32} \cdot Z_2}$$

Cette première manière de faire permet de déterminer le moment magnétique de l'objet magnétique 2. La précision de détermination ne dépend pas ici de la position relative de l'objet par rapport aux capteurs 32, 33.

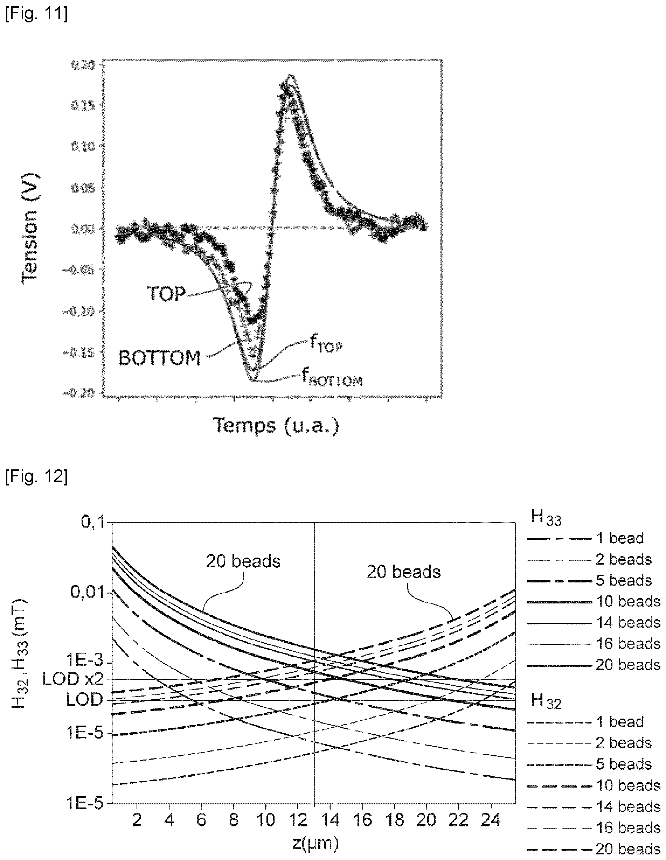

La [Fig. 11] montre une autre façon de réaliser le calcul 13 du moment magnétique $\mu$ de l'objet magnétique 2. Dans ce calcul, les signaux électriques TOP, BOTTOM mesurés aux bornes des deux capteurs 32, 33 sont pris en compte. Deux courbes théoriques $f_{TOP}$, $f_{BOTTOM}$ sont ajustées sur les deux signaux TOP, BOTTOM. L'étendue temporelle des signatures caractéristiques dépend en grande partie de la vitesse de passage de l'objet magnétique 2 entre les deux capteurs 32, 33 et donc de la vitesse d'écoulement, qui est connue. En revanche, l'amplitude des courbes TOP, BOTTOM en tout instant dépend du moment magnétique $\mu$ porté par l'objet magnétique 2. L'ajustement des courbes théoriques $f_{TOP}$, $f_{BOTTOM}$ dépend donc uniquement du moment magnétique $\mu$ de l'objet magnétique 2. La prise en compte des valeurs mesurées par les deux capteurs 32, 33 ainsi que l'ajustement des valeurs mesurées permettent d'améliorer davantage la détermination du moment magnétique $\mu$.

Les courbes théoriques $f_{TOP}$, $f_{BOTTOM}$ prennent en compte une estimation du champ magnétique $H$ rayonné par l'objet magnétique 2 et perçu par un capteur. Par exemple, le champ $H_{32}$ perçu au niveau du capteur top 32 et rayonné par un objet magnétique 2 aux coordonnées $x_2$, $y_2$ et $z_2$ est :

$$
\begin{aligned}
H_{32} = \Bigg( &\left(\frac{y_l}{q_2^2}\left(\frac{x_r}{r_2}-\frac{x_l}{r_1}\right)+\frac{y_r}{q_4^2}\left(\frac{x_l}{r_4}-\frac{x_r}{r_3}\right)\right)\sin\theta\sin\psi \\
&+\left(\frac{1}{r_1}-\frac{1}{r_2}+\frac{1}{r_3}-\frac{1}{r_4}\right)\sin\theta\cos\psi \\
&+\left(\frac{h}{q_2^2}\left(\frac{x_l}{r_1}-\frac{x_r}{r_2}\right)+\frac{h}{q_4^2}\left(\frac{x_r}{r_3}-\frac{x_l}{r_4}\right)\right)\cos\theta \Bigg)
\end{aligned}
$$

avec :

$$x_r = \frac{L_{32}}{2} - x_2 \qquad x_l = -\frac{L_{32}}{2} - x_2$$

$$y_r = \frac{l_{32}}{2} - y_2 \qquad y_l = -\frac{l_{32}}{2} - y_2$$

$$h = z_2 - z_{32}$$

$$r_1 = \sqrt{x_l^2 + y_l^2 + h^2} \quad r_2 = \sqrt{x_r^2 + y_l^2 + h^2}$$

$$r_3 = \sqrt{x_r^2 + y_r^2 + h^2} \quad r_4 = \sqrt{x_l^2 + y_r^2 + h^2}$$

$$q_2 = \sqrt{y_l^2 + h^2} \qquad q_4 = \sqrt{y_r^2 + h^2}$$

où $L_{32}$ et $l_{32}$ sont respectivement la largeur mesurée selon X et la longueur mesurée selon Y du capteur top 32, $\psi$ est l'angle entre $\mu$ et X, et $\theta$ est l'angle entre $\mu$ et Z. Lorsque le moment magnétique $\mu$ est aligné selon Z alors, le champ $H_{32}$ mesuré au niveau du capteur top 32 devient :

$$H_{32} = \frac{h}{q_2^2}\left(\frac{x_l}{r_1} - \frac{x_r}{r_2}\right) + \frac{h}{q_4^2}\left(\frac{x_r}{r_3} - \frac{x_l}{r_4}\right)$$

Le champ $H_{33}$ perçu au niveau du deuxième capteur 33 et émis par l'objet magnétique 2 aux coordonnées $x_2$, $y_2$ et $z_2$ peut être obtenu de la même manière.

La connaissance de la hauteur de passage $z_2$ et l'ajustement des champs magnétiques $H_{32}$ et $H_{33}$, représentés par les courbes $f_{TOP}$ et $f_{OTTOM}$ dans la [Fig. 12], permettent ainsi de déterminer le moment magnétique $\mu$ avec précision.

La [Fig. 1] montre un exemple de courbe de référence R($z_b$). Afin que les résultats obtenus à partir de cette courbe soient précis, il est préférable que la courbe R prenne en compte des caractéristiques de la biopuce 3. Pour cela, elle est par exemple générée en considérant un système de calibration. Le système de calibration comprend par exemple des capteurs de champ magnétique de calibration et un objet magnétique de calibration. Le système de calibration est par exemple un modèle, par exemple numérique. Il peut comprendre des capteurs de calibration qui sont par exemple des modèles des capteurs top et bottom 32, 33. Les capteurs de calibration sont par exemple établis à partir des positions relatives des capteurs top et bottom 32, 33 entre eux et par rapport au canal microfluidique 31. Ils peuvent également présenter des sensibilités de calibration, qui sont préférentiellement égales aux sensibilités des capteurs top et bottom 32, 33. Le système de calibration peut également comprendre un objet de calibration. Cet objet peut présenter le même diamètre que l'objet magnétique 2 que l'on souhaite caractériser au moyen de la biopuce 3 (on parlera alors d'objet magnétique cible). Il peut également présenter le même moment magnétique que l'objet magnétique 2 cible. Toutefois, il est avantageux que l'objet magnétique de calibration présente des caractéristiques indépendantes des caractéristiques des objets magnétiques 2 cibles. De cette manière, la courbe de référence peut être utilisée pour différentes objets magnétiques, quelque soit leur moment magnétique, voir leur diamètre. L'indépendance de l'objet de calibration quant au moment magnétique de l'objet magnétiques 2 cible est possible grâce à la prise en compte de rapport d'amplitudes plutôt que des valeurs absolues. Les rapports d'amplitudes sont indépendants des moments magnétiques considérés. Donc l'objet magnétique de calibration peut présenter un moment magnétique égal à 1.

Dans l'exemple de la [Fig. 1], la courbe de référence R(zb) correspond à un rapport du champ dipolaire maximum émis par l'objet de calibration (présentant un moment magnétique arbitraire) et perçu au niveau des capteurs de calibration. Dans cet exemple les capteurs de calibration sont distants d'une distance de 26 $\mu$m, mesurée selon la direction normale. Cette distance correspond ainsi à une biopuce 3 comprenant un canal microfluidique 31 présentant une hauteur hcan = 20 $\mu$m (seules cette plage de hauteurs est illustrée dans la [Fig. 1] puisque l'objet cible 2 ne peut pas pénétrer dans les parois du canal 31) et dont les capteurs top et bottom 32, 33 sont distants du canal 31 d'une distance respectivement égale à 5 $\mu$m et 1,7 $\mu$m. Dans cet exemple, le rayon Ro de l'objet cible 2 a été pris en compte (mais ce n'est pas obligatoire), ce qui permet de limiter la plage de hauteurs de passage $z_b$ à considérer. Elle s'étend en effet entre Ro et hcan-Ro.

Le procédé 1 comprend avantageusement une étape de détermination 10 de la courbe de référence. Selon un mode de réalisation, la courbe de référence R(zb) correspond à un rapport du champ dipolaire maximum émis par l'objet de calibration (présentant un moment magnétique arbitraire) et perçu au niveau des capteurs de calibration. Le calcul des champs perçus, tel que décrit précédemment (en référence aux champs $H_{32}$ et $H_{33}$), peut être considéré. Il s'agit en l'occurrence du calcul du champ émis par l'objet de calibration présentant un moment magnétique arbitraire au niveau des capteurs de calibration.

Le calcul est réalisé pour différentes hauteurs de passage z de l'objet magnétique de calibration entre les capteurs de calibration. Pour chaque hauteur de passage z, le champ magnétique dipolaire émis par l'objet magnétique de calibration et perçu par chaque capteur de calibration est déterminé.

La [Fig. 12] montre par exemple, un calcul des champs magnétiques maximum perçus par les capteurs de calibration (noté $H_{32}$ et $H_{33}$) et rayonnés par un objet magnétique de calibration présentant différents moments magnétiques. Le moment magnétique est donné en fonction d'un nombre de bille magnétique plutôt qu'en unité standard (c'est-à-dire en A·m$^2$). Chaque bille magnétique présente par exemple un moment magnétique égal à $1,56{\times}10^{-14}$ A·m$^2$. Le calcul est réalisé pour un nombre de bille compris entre 1 bille magnétique et 20 billes magnétiques.

Le champ magnétique maximum perçu par un premier capteur de calibration est matérialisé en traits pleins alors que le champ magnétique maximum perçu par un deuxième capteur de calibration est matérialisé en traits discontinus. Le champ magnétique maximum perçu croît ou décroît en fonction de la hauteur de passage de l'objet magnétique.

Le calcul du rapport des champs magnétiques maximum en fonction de la hauteur de passage permet d'obtenir la courbe de référence, telle que celle illustrée par la [Fig. 1]. Le rapport calculé est constant en fonction du nombre de billes magnétiques considéré.

Le calcul de la [Fig. 12] revêt un double intérêt en ce qu'il permet de prendre en compte une limite de détection théorique LOD (pour « Limite Of Detection » en anglais) des appareils électroniques. En deçà de la limite de détection théorique, on peut considérer que l'objet magnétique ne sera pas détecté. Ainsi, on peut déterminer le moment magnétique minimum (en l'occurrence le nombre de billes minimum) pour permettre une bonne caractérisation de l'objet cible 2.

Le rapport R des amplitudes de la courbe de référence peut être calculé à partir de champ magnétique perçus au niveau des capteurs de calibration. Lorsque les sensibilités des capteurs top et bottom 32, 33 sont identiques, alors le rapport des amplitudes des signaux électriques synchronisés est égal au rapport R de la courbe de référence. En revanche, les capteurs (réels) top et bottom 32, 33 peuvent avoir des sensibilités différentes. Le rapport R prend alors préférentiellement la sensibilité des capteurs top et bottom 32, 33 en compte. Pour cela, le champ magnétique perçu au niveau de chaque capteur de calibration est multiplié par la sensibilité du capteur de calibration en question et préférentiellement par la sensibilité du capteur top ou bottom 32, 33 qu'il modélise.

Les facteurs de sensibilité des capteurs top et bottom 32, 33 sont préférentiellement linéaires et préférentiellement compris entre 1 /mT et 2 %/mT.

Lorsque l'objet magnétique est un objet biologique marqué, au moyen de billes magnétiques, il est alors avantageux que le procédé 1 comprenne une étape 14 de calcul du nombre de billes magnétiques associé audit objet biologique. Le calcul est réalisé à partir du moment magnétique $\mu$ précédemment obtenu et à partir du moment magnétique d'une seule bille magnétique. Par exemple, des billes magnétique de la marque Dynabeads(TM) présente un moment magnétique $\mu$s $= 1,56{\times}10^{-14}$ A·m$^2$. Le moment magnétique d'une seule bille magnétique peut être déduit d'une mesure de moment magnétique d'une assemblée de billes, par exemple au moyen d'un magnétomètres à échantillons vibrants (dit « VSM » pour « Vibrating Sample Magnetometer » en anglais). Le nombre N de bille peut alors être obtenu par N $= \mu/\mu_B$.

L'étape de réception 11 des signaux électriques issus des capteurs 32, 33 peut comprend une première sous-étape 11a de filtrage des signaux reçus. Le filtrage consiste en une moyenne glissante sur chaque signal. La moyenne glissante présente préférentiellement une fenêtre d'environ 0,1 ms.

L'étape de réception 11 peut également comprendre une sous-étape d'identification 11b des signatures caractéristiques de la mesure d'un objet magnétique. La [Fig. 7] montrent un exemple de signatures caractéristiques. Chaque signature comprend un pic positif immédiatement suivi d'un pic négatif. Une inversion de sensibilité d'un des capteurs 32, 33 peut inverser cette signature (qui devient un pic négatif suivi d'un pic positif), tel qu'illustré par la [Fig. 8]. Dans une telle éventualité, le signal est préférentiellement inversé de sorte que les signatures de la mesure d'un objet présentent un pic positif suivi d'un pic négatif.

Les signatures caractéristiques sont, dans un premier temps, séparées du bruit de mesure. Pour cela, une sélection à partir de critère de forme peut être mise en œuvre. Par exemple, seuls les pics présentant une amplitude en valeur absolue supérieure à un seuil peuvent être considérés. En effet, une amplitude minimale, tant positive que négative, est attendue de la part des pics d'une signature caractéristique d'un objet magnétique 2. Ceci revient à considérer des plages de tension, par exemple représentées par les parties grisées autour de 0 V dans la [Fig. 9], comme comprenant principalement du bruit de mesure. Les pics considérés présentent une proéminence $P_{TOP}$, $P_{BOTTOM}$, mesurée hors des parties grisées, strictement supérieure à zéro. L'étendue des plages de tensions peut être déterminée en fonction de la densité de bruit de l'installation (mesuré sans l'introduction des objets magnétiques ciblés dans le canal microfluidique 3). Le bruit peut présenter une tension crête à crête $V_b$ auquel cas les plages ignorées sont égales $[-V_b ; V_b]$. Grâce à cette condition, les signaux générés par des billes magnétiques seules sont ignorées (notamment car le champ dipolaire qu'elles émettent donne lieu à des signaux dont l'amplitude est inférieure ou égale à la LOD).

Selon un autre exemple, compatible avec l'exemple précité, seuls les pics présentant des polarités alternées sont considérés. Il s'agit par exemple d'un pic positif suivi d'un pic négatif ou d'un pic négatif suivi d'un pic positif. Il est attendu que l'intervalle de temps séparant les amplitudes extrémales de ces deux pics soit inférieure à une durée prédéterminée. La durée prédéterminée varie en fonction de la taille de l'objet magnétique et sa vitesse de passage (la vitesse d'écoulement). Pour un objet présentant un diamètre de 10 $\mu$m dans un écoulement de 8 cm/s, la durée prédéterminée est comprise entre 100 $\mu$s et 600 $\mu$s.

Afin de faciliter l'identification des signatures caractéristiques, il est préférable que les signatures de plusieurs objets magnétiques ne se chevauchent pas. Pour cela, il est préférable que les objets magnétiques 2 soient suffisamment dilués dans la matrice fluide qui permet leur circulation dans le canal microfluidique 31.

Deux objets biologiques marqués donnent lieu à des signaux qui ne se confondent pas lorsqu'ils sont séparés de plus de 30 $\mu$m. Considérant un espacement $z32 - z33$ entre les deux capteurs 32, 33, il est préférable que la dilution des objets magnétiques permette d'avoir un espacement entre deux objets supérieur ou égal à $1,7 \times (z32 - z33)$. Ainsi ce procédé 1 est adapté à du diagnostic précoce puisque l'agrégation des objets biologiques satisfait ces deux critères.

L'étape de réception 11 peut également comprendre une sous-étape d'identification 11c des signaux électriques synchronisés entre les signatures caractéristiques du signal issu du capteur top 32 et les signatures caractéristiques du signal issu du capteur bottom 33. Deux signatures caractéristiques synchronisée correspondent à deux mesures d'un même objet magnétique 2. La synchronisation des deux signatures caractéristiques est alors appelée « coïncidence ».

Lors de la sous-étape d'identification des coïncidences, un premier instant est choisi au sein d'une signature caractéristique du premier signal et un deuxième instant est choisi au sein d'une signature caractéristique du deuxième signal. Le décalage temporel $t_{SYN}$ entre ces deux instants est mesuré. Lorsque les capteurs 32, 33 ne sont pas alignés, les signatures synchronisées peuvent présenter un décalage temporel intrinsèque, qui correspond à la distance séparant les deux capteurs 32, 33 selon la direction Y, multipliée par la vitesse de l'écoulement dans le canal 31. On peut donc déterminer une gamme $W_{SYN}$ de décalage temporel $t_{SYN}$ acceptable. Par exemple, pour un écoulement de 8 cm/s, on peut attendre que la gamme $W_{SYN}$ de décalage temporel $t_{SYN}$ s'étende sur 100 $\mu$s. Les signatures caractéristiques dont le décalage temporel est compris dans cette gamme sont alors dits synchronisés. Par exemple, lorsque les capteurs 32, 33 sont alignés (sans aucun décalage selon Y), le décalage temporel intrinsèque est nul et le décalage entre les deux signatures est alors compris dans la gamme de 100 $\mu$s (c'est-à-dire présentant un écart relatif de plus ou moins 50 $\mu$s).

Les premier et deuxième instants correspondent avantageusement à des caractéristiques comparables de chaque signature. Par exemple, lorsque le premier instant correspond au maximum (positif) de la signature caractéristique du premier signal, alors le deuxième instant correspond alors avantageusement au maximum (positif) de la signature caractéristique du deuxième signal.

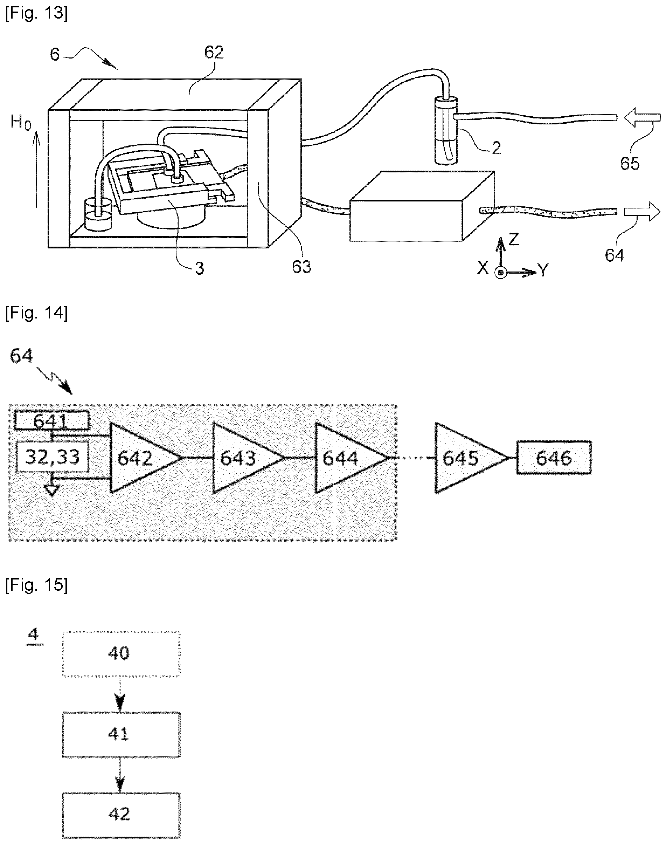

La [Fig. 13] représente schématiquement un dispositif 6 de détermination 1 du moment magnétique $\mu$ d'objets magnétiques 2. Le dispositif 6 comprend la biopuce 3 telle que décrite précédemment. Il comprend également des moyens complémentaires 62, 64, 65, adaptés pour mettre en œuvre le procédé de détermination 1 selon l'invention.

Le dispositif 6 comprend un moyen 62 d'application d'un champ magnétique $H_0$ sur la biopuce 3. Le moyen 62 comprend un aimant permanent 62 configuré pour appliquer le champ $H_0$ au niveau de la biopuce 3. Le champ $H_0$ est préférentiellement aligné selon la direction Z. Ce champ H0 permet par exemple d'aligner le moment magnétique $\mu$ des objets magnétiques selon la direction Z (tel qu'illustré par la [Fig. 6]). De la sorte, la capacité de détection des capteurs 32, 33 est maximale.

Un fer doux 63 peut être ajouté pour isoler la biopuce de l'environnement extérieur (et notamment du champ magnétique extérieur). Ce blindage 63 permet d'homogénéiser le champ magnétique $H_0$ dans lequel baigne la biopuce 3 et également améliorer le rapport de signal sur bruit des capteurs 32, 33.

Les objets magnétiques 2 à caractériser sont préférentiellement mélangé dans une matrice fluide. De la sorte, la matrice fluide et les objets magnétiques 2 peuvent être mis en circulation dans le canal microfluidique 31 de la biopuce 3. Le dispositif 6 comprend un système de contrôle 65 de la vitesse d'écoulement de la matrice fluide dans laquelle baignent les objets magnétiques 2.

Le dispositif 6 comprend également une chaîne d'acquisition 64 chargé de polariser les capteurs 32, 33 (par exemple en courant) et réceptionner les signaux électriques issue des capteurs 32, 33.

La [Fig. 14] montre un exemple de chaîne d'acquisition 64. Elle comprend notamment une source de courant 641 chargée de polariser les capteurs 32, 33 en courant. Un voltmètre 642 permet de mesurer la tension aux bornes de chaque capteurs 32, 33. Le voltmètre 642 comprend avantageusement un étage d'amplification, par exemple de + 60 dB, afin de fournir des valeurs de tension exploitables. Le voltmètre peut également comprendre un filtre passe-haut, afin de supprimer le bruit de très basse fréquence, par exemple inférieure à 150 Hz. La chaîne d'acquisition 64 peut également comprendre un étage d'amplification supplémentaire 643. Elle peut également comporter un filtre passe-bas 644, chargé de couper les très hautes fréquences, par exemple supérieure à 37 kHz.

La chaîne 64 peut également comprendre un convertisseur numérique 645 et un contrôleur 646, chargé de traiter les signaux électriques issus des capteurs 32, 33. La fréquence d'échantillonnage du convertisseur 645 dépend en partie de la vitesse d'écoulement. Par exemple, pour une vitesse d'écoulement, la fréquence d'échantillonnage est préférentiellement supérieure ou égale à 200 kHz.

Le contrôleur 646 permet par exemple de réaliser les étapes de calcul 12, 13, 14 du procédé 1.

La détermination 1 du moment magnétique $\mu$ des objets magnétiques 2 passant dans le canal microfluidique 31 peut être réalisée en « temps réel », c'est-à-dire simultanément au passage des objets magnétiques 2 entre les capteurs ou alors en différé. Dans ce dernier cas, les signaux mesurés TOP, BOTTOM par les capteurs 32, 33 sont enregistrés dans des fichiers et traité dans un second temps.

La [Fig. 15] illustre schématiquement un procédé de procédé 4 de comptage d'objets biologiques 5 mettant en œuvre la biopuce 3 et le procédé détermination d'un moment magnétique, tels que décrit précédemment.

Les objets biologiques 5 sont par exemple des cibles biologiques de tailles variées, telles que des cellules, des bactéries ou encore des virus. Il s'agit par exemple de cellules cancéreuse ou de bactéries pathogènes : salmonelles, yersinia enterocolitica ou encore legionella pneumophile.

Excepté les magnétosomes qui possèdent un cœur magnétique intrinsèque, les objets biologiques 5 ne sont pas magnétiques. Il est alors nécessaire de réaliser un marquage de ces derniers permettant de leur adjoindre un moment magnétique. De la sorte, ils peuvent être comptés au moyen d'un procédé basé sur une mesure d'un moment magnétique.

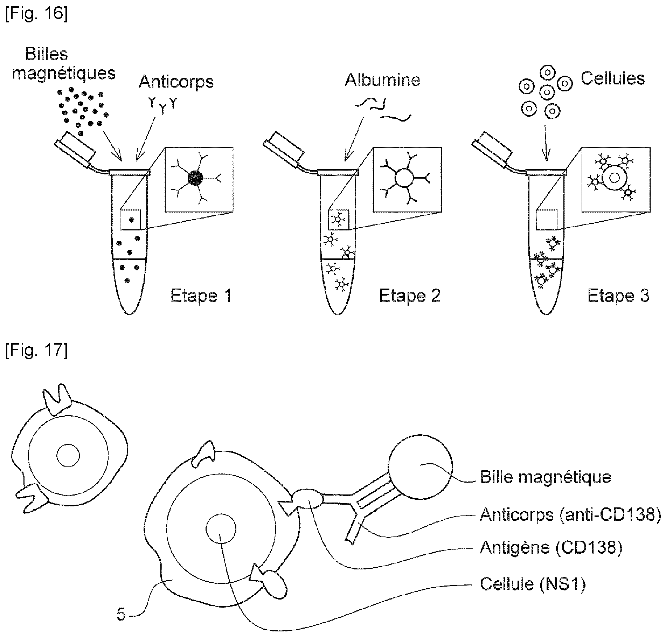

La [Fig. 16] illustre schématiquement une étape de marquage d'objet biologiques. Pour cela, le procédé 4 de comptage comprend dans un premier temps une étape de marquage 41 des objets biologiques 5. Pour ce faire, les objets biologiques 5, ici des cellules, sont mélangée à une matrice comprenant des billes magnétiques. Les billes magnétiques sont aptes à marquer les objets biologiques 5. Elles sont par exemple associées à des anticorps capables de se coupler avec des antigènes portés par les objets biologiques 5.

Parmi les billes magnétiques (de tailles variant par exemple de 200 nm à 1 $\mu$m), trois types de fonctionnalités de surface existent. Les groupes ester sulfonique et acide carboxylique réagissent chimiquement avec les anticorps et créent des liaisons covalentes tandis que les billes-streptavidine sont fonctionnalisées via l'interaction biotine-streptavidine par des anticorps préalablement couplés de façon covalente à de la biotine.

Par exemple, dans une première étape, des billes magnétiques de taille variant de 200 nm à 1 $\mu$m et des anticorps sont mélangés dans une solution neutre. Le protocole est spécifique pour chacun des lots de billes étudié. Les billes magnétiques sont par exemple des billes micrométriques constituées de cœurs d'oxyde de fer (maghémite ou magnétite) de taille nanométrique enveloppés dans une couche de polymère. Les anticorps se fixent sur la couche polymère des billes magnétiques

Les billes fonctionnalisées par les anticorps sont ajoutées dans la matrice contenant les objets biologiques 5. Elles sont ajoutées en excès afin de favoriser la probabilité de rencontre avec les objets biologiques.

Une partie des anticorps va s'associer avec des antigènes portés par chaque cellule 5. De la sorte, les cellules 5 sont dites « marquées », c'est-à-dire fonctionnalisées au moyen de billes magnétiques. Les cellules 5 marquées portent par exemple entre 50 et 100 billes magnétiques de diamètre 1 $\mu$m. Malgré les précautions apportées, une partie des cellules 5, de l'ordre de 7%, peuvent restées non marquées. D'ailleurs, plus les cellules 5 marquées sont grosses et plus elles sont susceptibles de porter un nombre élevé de billes magnétiques, ce qui peut faciliter leur comptage.

La [Fig. 17] montre un exemple d'objet biologique 5 marqué, en l'occurrence une cellule comprenant des antigènes dont l'un deux est associé à un anticorps. Une bille magnétique est fixée sur l'anticorps en question. L'objet biologique 5 marqué est, grâce à la bille magnétique, porteur d'un moment magnétique $\mu$.

Le procédé de comptage de la [Fig. 15] comprend également une étape de détermination du moment magnétique $\mu$ d'objets magnétiques passant entre les capteurs 32, 33. Lors de cette étape, la matrice comprenant les objets biologiques 5 marqués circule dans le canal microfluidique 31 de la biopuce 3 (telle que décrite précédemment). Les signaux électriques des capteurs 32, 33 sont acquis à mesure que les objets biologiques 5 circulent dans le canal 31. Pour chaque coïncidence, telle que décrite précédemment, un moment magnétique $\mu$ est calculé.

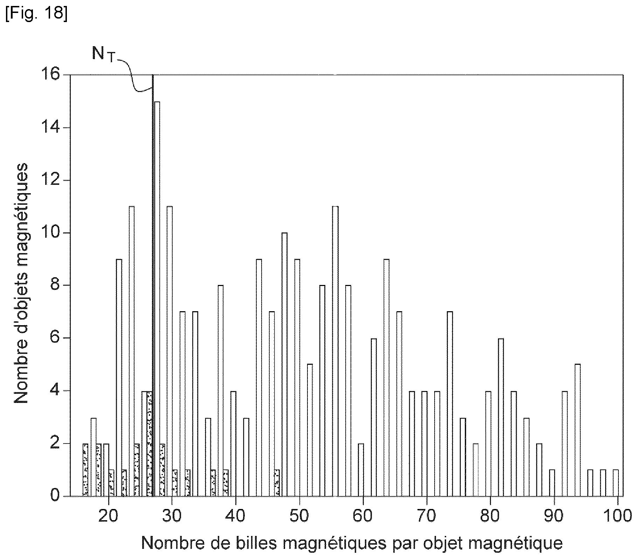

La [Fig. 18] illustre par exemple un diagramme de répartition des objets magnétiques 2 détectés en fonction du nombre de billes magnétiques que possèdent les objets magnétiques détectés. L'abscisse de la figure indique un nombre de billes magnétiques par objet magnétique caractérisé plutôt que la valeur du moment magnétique. Le nombre de billes magnétiques est déterminée comme le rapport du moment magnétique de l'objet détecté et le moment magnétique individuel de la bille magnétique (déterminé de manière préliminaire).

Les barres blanches dans la [Fig. 18] correspondent à un comptage réalisé dans un échantillon spécifique comprenant des objets biologiques d'intérêt marqués dans une matrice complexe (milieu de culture). Les barres noires correspondent en revanche à un échantillon dit « contrôle négatif » contenant la même matrice complexe (avec le même nombre de billes magnétiques fonctionnalisées) mais sans objet biologique marqué (il s'agit en l'occurrence du sous-produit de l'étape 2 de la [Fig. 17]).

Dans ce dernier cas (barres noires), des objets magnétiques sont détectés et présentent un moment magnétique. Il ne s'agit en revanche pas d'objets biologiques cibles (puisque absents de la matrice pour réaliser spécifiquement ces mesures). Il s'agit d'agrégats magnétiques qui peuvent être confondus avec des objets biologiques marqués. Ils sont donc à différencier des objets biologiques 5 marqués. Ces agrégats magnétiques comprennent entre deux et vingt-cinq billes magnétiques par agrégats. Ils ne peuvent être détectés qu'à partir de quatre billes magnétiques quand ils passent au centre et à partir de douze billes sur la totalité du canal. En dessous de quatre billes magnétiques, les agrégats émettent un champ dipolaire qui est généralement inférieur à la limite de détection (dite « LOD »).

Afin d'éviter de comptabiliser les agrégats magnétiques, un moment magnétique seuil est considéré. Dans l'exemple de la [Fig. 18], il correspond à un nombre de billes magnétiques seuil, égal, dans l'exemple proposé, à vingt-cinq billes.

Dès lors, le procédé de comptage 4 peut comptabiliser chaque objet magnétique pour un objet biologique marqué si le moment magnétique de l'objet magnétique (et donc le nombre de bille magnétiques qu'il porte) et supérieur au moment magnétique seuil (c'est-à-dire supérieur au nombre de bille magnétiques seuil $N_T$). De la sorte, les agrégats magnétiques ne sont pas comptabilisés.

## Revendications

1. Procédé (1) de détermination du moment magnétique ($\mu$) d'un objet magnétique (2) au moyen d'une biopuce (3), l'objet magnétique étant un objet biologique (5) marqué au moyen de billes magnétiques ou un magnétosome ou un agrégat de billes magnétiques, la biopuce comprenant : un canal microfluidique (31) s'étendant dans un plan (P) et présentant une hauteur (hcan), mesurée selon une direction (Z) dite « direction normale » perpendiculaire au plan (P) ; et deux capteurs de champ magnétique (31, 32), disposés de part et d'autre du canal microfluidique (31), le procédé (1) comprenant les étapes suivantes :

   - recevoir (11) deux signaux électriques synchronisés (TOP, BOTTOM), issus respectivement des capteurs de champ magnétique (32, 33) correspondant au passage de l'objet magnétique (2) dans le canal microfluidique (31) au niveau de chaque capteur de champ magnétique (32, 33) ;
   - calculer (12) la hauteur de passage ($z_2$) de l'objet magnétique (2), mesurée selon la direction normale (Z), à partir du rapport des amplitudes des signaux électriques synchronisés (TOP, BOTTOM) et au moyen d'une courbe de référence ($R(z_b)$) reliant la hauteur de passage ($z_b$) d'un objet magnétique de calibration dans le canal microfluidique (31) avec un rapport (R) des amplitudes de signaux électriques de calibration correspondant au passage de l'objet magnétique de calibration à ladite hauteur de passage ($z_b$), ledit rapport (R) des amplitudes de signaux électriques de calibration augmentant de manière non-linéaire à mesure que la hauteur de passage ($z_b$) de l'objet magnétique de calibration augmente jusqu'à atteindre une valeur maximale, le rapport des amplitudes des signaux électriques synchronisés (TOP, BOTTOM) étant égal au rapport (R) des amplitudes de signaux électriques de calibration,
   - calculer (13) le moment magnétique ($\mu$) de l'objet magnétique (2) à partir de la hauteur de passage ($z_2$) déterminée et d'un des signaux électriques synchronisés.

2. Procédé (1) selon la revendication précédente, dans lequel la courbe de référence ($R(z_b)$) est déterminée à partir de l'objet magnétique de calibration présentant un moment magnétique de calibration indépendant du moment magnétique de l'objet magnétique.

3. Procédé (1) selon l'une des revendications précédentes, dans lequel le signal électrique (TOP, BOTTOM) issu de chaque capteur de champ magnétique (32, 33) est égal au champ dipolaire ($H_{dip}$) émis par l'objet magnétique (2) multiplié par un facteur de sensibilité du capteur de champ magnétique (32, 33).

4. Procédé (1) selon la revendication précédente, dans lequel chaque signal électrique de calibration est égal au champ dipolaire émis par l'objet magnétique de calibration au niveau d'un capteur de champ magnétique de calibration multiplié par un facteur de sensibilité de calibration, les facteurs de sensibilité de calibration étant égaux aux facteurs de sensibilité des deux capteurs de champ magnétique (32, 33).

5. Procédé (1) selon l'une des revendications précédentes, comprenant une étape de détermination (10) de la courbe de référence comprenant les sous-étapes suivantes :

   - pour différentes hauteurs de passage de l'objet magnétique de calibration entre deux capteurs de champ magnétique de calibration :

     o déterminer le champ magnétique dipolaire émis par l'objet magnétique de calibration et perçu par l'un des capteurs de champ magnétique de calibration ;
     o déterminer le champ magnétique dipolaire émis par l'objet magnétique de calibration et perçu par l'autre des capteurs de champ magnétique de calibration ;

   - calculer le rapport des amplitudes des champs magnétiques dipolaires perçus par les capteurs de champ magnétique de calibration en fonction des différentes hauteur de passage de l'objet magnétique de calibration.

6. Procédé (1) selon l'une des revendications précédentes, dans lequel l'étape de réception (11) comprend également une sous-étape d'identification (11c) des signaux électriques synchronisés comprenant la mesure d'un écart temporel ($t_{SYN}$) entre des signatures caractéristiques des deux signaux électriques (TOP, BOTTOM), chaque signature caractéristique correspondant à la mesure de l'objet magnétique (2) par l'un des capteurs de champ magnétique (32, 33), la synchronisation étant identifiée lorsque l'écart temporel ($t_{SYN}$) est compris dans une gamme temporelle prédéterminée ($W_{SYN}$).

7. Procédé (1) selon l'une des deux revendications précédentes, dans lequel l'étape de réception (11) comprend une sous-étape d'identification (11b) des signatures caractéristiques dans chacun des signaux électriques à partir de critères de forme des signaux électriques.

8. Procédé (1) selon l'une des revendications précédentes, dans lequel l'objet magnétique est un objet biologique marqué au moyen de billes magnétiques, ledit procédé (1) comprenant également une étape de calcul (14) du nombre de billes magnétiques associé à l'objet biologique (5) marqué à partir du moment magnétique calculé et du moment magnétique d'une seule bille magnétique.

9. Procédé (1) selon l'une des revendications précédentes, dans lequel les capteurs (32, 33) de champ magnétique sont des capteurs magnétorésistifs.

10. Procédé (4) de comptage d'objets biologiques (5) comprenant les étapes suivantes :

 - marquer (41) les objets biologiques (5) en les mélangeant à une matrice liquide comprenant des billes magnétiques aptes à s'accrocher à des récepteurs portés par chaque objet biologique ;
 - faire circuler la matrice liquide comprenant les objets biologiques (5) marqués dans le canal microfluidique (31) de la biopuce (3) et déterminer (42) le moment magnétique ($\mu$) d'objets magnétiques (2) passant entre les capteurs de champ magnétique (32, 33) au moyen du procédé (1) selon l'une des revendications 1 à 9, chaque objet magnétique (2) comptant pour un objet biologique (5) marqué lorsque que le moment magnétique ($\mu$) qui lui ait associé est supérieur à un moment magnétique seuil (NT).

11. Procédé (4) de comptage selon la revendication précédente comprenant l'étape de déterminer (40) le moment magnétique seuil ($N_T$), ladite étape comprenant les sous-étapes suivantes :

 - faire circuler la matrice liquide sans objet biologique, dite « contrôle négatif », dans le canal microfluidique de la biopuce (3) et déterminer le moment magnétique ($\mu$) d'objets magnétiques (2) passant entre les capteurs de champ magnétique au moyen du procédé (1) de détermination du moment magnétique selon l'invention ; et
 - fixer le moment magnétique seuil (NT) à partir d'une répartition statistique des moments magnétiques déterminés.

12. Dispositif (6) de détermination du moment magnétique ($\mu$) d'un objet magnétique (2), l'objet magnétique étant un objet biologique (5) marqué au moyen de billes magnétiques ou un magnétosome ou un agrégat de billes magnétiques, comprenant :

 - une biopuce comprenant : un canal microfluidique (31) s'étendant dans un plan (P) et présentant une hauteur (hcan), mesurée selon une direction (Z) dite « direction normale » perpendiculaire au plan (P) ; et deux capteurs de champ magnétique (31, 32), disposés de part et d'autre du canal microfluidique (31),
 - un aimant (62) configuré pour appliquer un champ magnétique (Ho) au niveau de la biopuce,
 - un système de contrôle de la vitesse d'écoulement d'une matrice fluide (65) configuré pour faire circuler une matrice liquide comprenant les objets biologiques (5) marqués dans le canal microfluidique (31) de la biopuce (3),
 - une chaîne d'acquisition (64) chargée de polariser les capteurs (32, 33) et de réceptionner les deux signaux électriques synchronisés (TOP, BOTTOM) issue desdits capteurs (32, 33), correspondant au passage de l'objet magnétique (2) dans le canal microfluidique (31) au niveau de chaque capteur de champ magnétique (32, 33) ; et comprenant un contrôleur configuré pour :

 ○ calculer (12) la hauteur de passage ($z_2$) de l'objet magnétique (2), mesurée selon la direction normale (Z), à partir du rapport des amplitudes des signaux électriques synchronisés (TOP, BOTTOM) et au moyen d'une courbe de référence ($R(z_b)$) reliant la hauteur de passage ($z_b$) d'un objet magnétique de calibration dans le canal microfluidique (31) avec un rapport (R) des amplitudes de signaux électriques de calibration correspondant au passage de l'objet magnétique de calibration à ladite hauteur de passage ($z_b$), ledit rapport (R) des amplitudes de signaux électriques de calibration augmentant de manière non-linéaire à mesure que la hauteur de passage ($z_b$) de l'objet magnétique de calibration augmente jusqu'à atteindre une valeur maximale, le rapport des amplitudes des signaux électriques synchronisés (TOP, BOTTOM) étant égal au rapport (R) des amplitudes de signaux électriques de calibration, et

∘ calculer (13) le moment magnétique ($\mu$) de l'objet magnétique (2) à partir de la hauteur de passage ($z_2$) déterminée et d'un des signaux électriques synchronisés.

**13.** Système de comptage d'objets biologiques (5), comprenant :

- des moyens pour marquer (41) les objets biologiques (5) en les mélangeant à une matrice liquide comprenant des billes magnétiques aptes à s'accrocher à des récepteurs portés par chaque objet biologique ;
- le dispositif de détermination du moment magnétique ($\mu$) d'un objet magnétique selon la revendication 12, dans lequel le contrôleur de la chaine d'acquisition est en outre configuré pour compter un objet biologique (5) marqué lorsque que le moment magnétique ($\mu$) qui lui est associé est supérieur à un moment magnétique seuil (NT).

**14.** Programme d'ordinateur comprenant des instructions qui conduisent le dispositif (6) selon la revendication 12 à exécuter les étapes du procédé de détermination (1) selon l'une des revendications 1 à 9.

**15.** Programme d'ordinateur comprenant des instructions qui conduisent le système de comptage selon la revendication 13 à exécuter les étapes du procédé (4) de comptage selon l'une des revendications 10 à 11.

**Patentansprüche**

**1.** Verfahren (1) zur Bestimmung des magnetischen Moments ($\mu$) eines magnetischen Objekts (2) mittels eines Biochips (3), wobei das magnetische Objekt ein mit Magnetkügelchen markiertes biologisches Objekt (5) oder ein Magnetosom oder ein Aggregat aus Magnetkügelchen ist, wobei der Biochip umfasst: einen Mikrofluidik-Kanal (31), der sich in einer Ebene (P) erstreckt und eine Höhe (hcan) aufweist, gemessen in einer Richtung (Z), die als "Normalenrichtung" bezeichnet wird und senkrecht zur Ebene (P) steht; und zwei Magnetfeldsensoren (31, 32), die zu beiden Seiten des Mikrofluidik-Kanals (31) angeordnet sind, wobei das Verfahren (1) die folgenden Schritte umfasst:

- Empfangen (11) von zwei synchronisierten elektrischen Signalen (TOP, BOTTOM), die jeweils von den Magnetfeldsensoren (32, 33) stammen und dem Durchgang des magnetischen Objekts (2) durch den Mikrofluidik-Kanal (31) auf Höhe jedes Magnetfeldsensors (32, 33) entsprechen;
- Berechnen (12) der Durchgangshöhe ($z_2$) des magnetischen Objekts (2), gemessen in der Normalenrichtung (Z), aus dem Verhältnis der Amplituden der synchronisierten elektrischen Signale (TOP, BOTTOM) und mittels einer Referenzkurve ($R(z_b)$), die die Durchgangshöhe ($z_b$) eines magnetischen Kalibrierungsobjekts im Mikrofluidik-Kanal (31) mit einem Verhältnis (R) der Amplituden der elektrischen Kalibrierungssignale in Verbindung bringt, die dem Durchgang des magnetischen Kalibrierungsobjekts in dieser Durchgangshöhe ($z_b$) entspricht, wobei das Verhältnis (R) der Amplituden der elektrischen Kalibrierungssignale nichtlinear zunimmt, wenn die Durchgangshöhe ($z_b$) des magnetischen Kalibrierungsobjekts zunimmt, bis ein Maximalwert erreicht ist, wobei das Verhältnis der Amplituden der synchronisierten elektrischen Signale (TOP, BOTTOM) gleich dem Verhältnis (R) der Amplituden der elektrischen Kalibrierungssignale ist,
- Berechnen (13) des magnetischen Moments ($\mu$) des magnetischen Objekts (2) anhand der bestimmten Durchgangshöhe ($z_2$) und eines der synchronisierten elektrischen Signale.

**2.** Verfahren (1) nach dem vorstehenden Anspruch, bei dem die Referenzkurve ($R(z_b)$) anhand des magnetischen Kalibrierungsobjekts bestimmt wird, das ein vom magnetischen Moment des magnetischen Objekts unabhängiges magnetisches Kalibrierungsmoment aufweist.

**3.** Verfahren (1) nach einem der vorstehenden Ansprüche, bei dem das von jedem Magnetfeldsensor (32, 33) ausgegebene elektrische Signal (TOP, BOTTOM) gleich dem vom magnetischen Objekt (2) ausgesandten Dipolfeld ($H_{dip}$) multipliziert mit einem Empfindlichkeitsfaktor des Magnetfeldsensors (32, 33) ist.

**4.** Verfahren (1) gemäß dem vorstehenden Anspruch, bei dem jedes elektrische Kalibrierungssignal dem Dipolfeld entspricht, das vom magnetischen Kalibrierungsobjekt an einem Kalibrierungsmagnetfeldsensor ausgesendet wird, multipliziert mit einem Kalibrierungsempfindlichkeitsfaktor, wobei die Kalibrierungsempfindlichkeitsfaktoren den Empfindlichkeitsfaktoren der beiden Magnetfeldsensoren (32, 33) entsprechen.

5. Verfahren (1) nach einem der vorstehenden Ansprüche, einen Schritt zur Bestimmung (10) der Referenzkurve umfassend, der die folgenden Teilschritte umfasst:

- für verschiedene Durchgangshöhen des magnetischen Kalibrierungsobjekts zwischen zwei Kalibrierungsmagnetfeldsensoren:

◦ Bestimmen des Dipolfelds, das vom magnetischen Kalibrierungsobjekt ausgesendet und von einem der magnetischen Kalibrierungssensoren wahrgenommen wird;
◦ Bestimmen des vom magnetischen Kalibrierungsobjekt ausgesendeten und vom anderen der magnetischen Kalibrierungssensoren wahrgenommenen Dipolfelds;

- Berechnen des Verhältnisses der Amplituden der von den magnetischen Kalibrierungssensoren wahrgenommenen Dipolfelder in Abhängigkeit von den verschiedenen Durchgangshöhen des magnetischen Kalibrierungsobjekts.

6. Verfahren (1) nach einem der vorstehenden Ansprüche, bei dem der Empfangsschritt (11) auch einen Teilschritt zur Identifizierung (11c) der synchronisierten elektrischen Signale umfasst, der die Messung einer Zeitabweichung (tsYN) zwischen charakteristischen Signaturen der beiden elektrischen Signale (TOP, BOTTOM) umfasst, wobei jede charakteristische Signatur der Messung des magnetischen Objekts (2) durch einen der Magnetfeldsensoren (32, 33) entspricht, wobei die Synchronisation identifiziert wird, wenn die Zeitabweichung ($t_{SYN}$) in einem vorbestimmten Zeitbereich ($W_{SYN}$) liegt.

7. Verfahren (1) nach einem der beiden vorstehenden Ansprüche, bei dem der Empfangsschritt (11) einen Teilschritt (11b) zum Identifizieren der charakteristischen Signaturen in jedem der elektrischen Signale anhand von Formkriterien der elektrischen Signale umfasst.

8. Verfahren (1) nach einem der vorstehenden Ansprüche, bei dem das magnetische Objekt ein biologisches Objekt ist, das mit Magnetkügelchen markiert ist, wobei das Verfahren (1) auch einen Schritt (14) zum Berechnen der Anzahl der dem markierten biologischen Objekt (5) zugeordneten Magnetkügelchen aus dem berechneten magnetischen Moment und dem magnetischen Moment eines einzelnen Magnetkügelchens umfasst.

9. Verfahren (1) nach einem der vorstehenden Ansprüche, bei dem die Magnetfeldsensoren (32, 33) magnetoresistive Sensoren sind.

10. Verfahren (4) zum Zählen biologischer Objekte (5), das die folgenden Schritte umfasst:

- Markieren (41) der biologischen Objekte (5) durch Mischen mit einer flüssigen Matrix, die Magnetkügelchen enthält, die sich an Rezeptoren anlagern können, die von jedem biologischen Objekt getragen werden;
- Zirkulierenlassen der flüssigen Matrix, die die markierten biologischen Objekte (5) enthält, in dem Mikrofluidik-Kanal (31) des Biochips (3) und Bestimmen (42) des magnetischen Moments ($\mu$) der magnetischen Objekte (2), die zwischen den Magnetfeldsensoren (32, 33) hindurchlaufen, mittels des Verfahrens (1) gemäß einem der Ansprüche 1 bis 9, wobei jedes magnetische Objekt (2) als markiertes biologisches Objekt (5) zählt, wenn das ihm zugeordnete magnetische Moment ($\mu$) größer ist als ein Schwellenwert für das magnetische Moment (NT).

11. Zählverfahren (4) gemäß dem vorstehenden Anspruch, das den Schritt des Bestimmens (40) des Schwellenwerts für das magnetische Moment (NT) umfasst, wobei dieser Schritt die folgenden Teilschritte umfasst:

- das Zirkulierenlassen der flüssigen Matrix ohne biologische Objekte, die sogenannte "Negativkontrolle", durch den Mikrofluidik-Kanal des Biochips (3) und Bestimmen des magnetischen Moments ($\mu$) der magnetischen Objekte (2), die zwischen den Magnetfeldsensoren hindurchlaufen, mittels des erfindungsgemäßen Verfahrens (1) zur Bestimmung des magnetischen Moments; und
- Festlegen des Schwellenwerts für das magnetische Moment (NT) anhand einer statistischen Verteilung der ermittelten magnetischen Momente.

12. Vorrichtung (6) zur Bestimmung des magnetischen Moments ($\mu$) eines magnetischen Objekts (2), wobei das magnetische Objekt ein mit Magnetkügelchen markiertes biologisches Objekt (5) oder ein Magnetosom oder ein Aggregat aus Magnetkügelchen ist, umfassend:

- einen Biochip, der umfasst: einen Mikrofluidik-Kanal (31), der sich in einer Ebene (P) erstreckt und eine Höhe (hcan) aufweist, gemessen in einer Richtung (Z), die als "Normalenrichtung" bezeichnet wird und senkrecht zur Ebene (P) steht; und zwei Magnetfeldsensoren (31, 32), die an beiden Seiten des Mikrofluidik-Kanals (31) angeordnet sind,
- einen Magneten (62), der so konfiguriert ist, dass er auf den Biochip ein Magnetfeld (Ho) ausübt,
- ein System zur Steuerung der Fließgeschwindigkeit einer flüssigen Matrix (65), das so konfiguriert ist, dass es eine flüssige Matrix, die die markierten biologischen Objekte (5) enthält, im Mikrofluidik-Kanal (31) des Biochips (3) zirkulieren lässt,
- eine Erfassungskette (64), die dafür zuständig ist, die Sensoren (32, 33) zu polarisieren und die beiden synchronisierten elektrischen Signale (TOP, BOTTOM) von den Sensoren (32, 33) zu empfangen, die dem Durchgang des magnetischen Objekts (2) im Mikrofluidik-Kanal (31) auf Höhe jedes Magnetfeldsensors (32, 33) entsprechen; und mit einer Steuereinheit, die konfiguriert ist zum:

o Berechnen (12) der Durchgangshöhe ($z_2$) des magnetischen Objekts (2), gemessen in der Normalenrichtung (Z), aus dem Verhältnis der Amplituden der synchronisierten elektrischen Signale (TOP, BOTTOM) und mittels einer Referenzkurve ($R(z_b)$), die die Durchgangshöhe ($z_b$) eines magnetischen Kalibrierungsobjekts im Mikrofluidik-Kanal (31) mit einem Verhältnis (R) der Amplituden der elektrischen Kalibrierungssignale in Verbindung bringt, das dem Durchgang des magnetischen Kalibrierungsobjekts in dieser Durchgangshöhe ($z_b$) entspricht, wobei das Verhältnis (R) der Amplituden der elektrischen Kalibrierungssignale nichtlinear zunimmt, wenn die Durchgangshöhe ($z_b$) des magnetischen Kalibrierungsobjekts zunimmt, bis ein Maximalwert erreicht ist, wobei das Verhältnis der Amplituden der synchronisierten elektrischen Signale (TOP, BOTTOM) gleich dem Verhältnis (R) der Amplituden der elektrischen Kalibrierungssignale ist, und
o Berechnen (13) des magnetischen Moments ($\mu$) des magnetischen Objekts (2) aus der bestimmten Durchgangshöhe ($z_2$) und einem der synchronisierten elektrischen Signale.

13. System zum Zählen biologischer Objekte (5), umfassend:

- Mittel zum Markieren (41) der biologischen Objekte (5) durch Mischen mit einer flüssigen Matrix, die magnetische Kügelchen enthält, die an Rezeptoren haften können, die von jedem biologischen Objekt getragen werden;
- die Vorrichtung zur Bestimmung des magnetischen Moments ($\mu$) eines magnetischen Objekts gemäß Anspruch 12, wobei die Steuerung der Erfassungskette außerdem so konfiguriert ist, dass sie ein markiertes biologisches Objekt (5) zählt, wenn das ihm zugeordnete magnetische Moment ($\mu$) größer als ein Schwellenwert für das magnetische Moment (NT) ist.

14. Computerprogramm mit Anweisungen, die die Vorrichtung (6) gemäß Anspruch 12 dazu veranlassen, die Schritte des Bestimmungsverfahrens (1) gemäß einem der Ansprüche 1 bis 9 auszuführen.

15. Computerprogramm mit Anweisungen, die das Zählsystem gemäß Anspruch 13 dazu veranlassen, die Schritte des Zählverfahrens (4) gemäß einem der Ansprüche 10 bis 11 auszuführen.

**Claims**

1. A method (1) for determining the magnetic moment ($\mu$) of a magnetic object (2) by means of a biochip (3), the magnetic object being a biological object (5) marked by means of magnetic beads or a magnetosome or an aggregate of magnetic beads, the biochip comprising: a microfluidic channel (31) extending in a plane (P) and having a height (hcan), measured along a direction (Z) referred to as the "normal direction" perpendicular to the plane (P); and two magnetic field sensors (31, 32), disposed on either side of the microfluidic channel (31), the method (1) comprising the following steps of:

- receiving (11) two synchronised electrical signals (TOP, BOTTOM) from the magnetic field sensors (32, 33) respectively, corresponding to the passage of the magnetic object (2) into the microfluidic channel (31) at each magnetic field sensor (32, 33);
- calculating (12) the passage height ($z_2$) of the magnetic object (2), measured along the normal direction (Z), from the ratio of the amplitudes of the synchronised electrical signals (TOP, BOTTOM) and by means of a reference curve ($R(z_b)$) relating the passage height ($z_b$) of a calibration magnetic object in the microfluidic channel (31) to a ratio (R) of the amplitudes of the calibration electrical signals corresponding to the passage of the calibration magnetic object at said passage height ($z_b$), said ratio (R) of the amplitudes of the calibration electrical signals non-linearly increasing as the passage height ($z_b$) of the calibration magnetic object increases until it reaches a maximum value, the ratio of the amplitudes of the synchronised electrical signals (TOP, BOTTOM) being equal to the ratio (R) of the amplitudes of the calibration electrical signals,
- calculating (13) the magnetic moment ($\mu$) of the magnetic object (2) from the passage height ($z_2$) determined and one of the synchronised electrical signals.

2. The method (1) according to the preceding claim, wherein the reference curve ($R(z_b)$) is determined from the calibration magnetic object having a calibration magnetic moment independent of the magnetic moment of the magnetic object.

3. The method (1) according to one of the preceding claims, wherein the electrical signal (TOP, BOTTOM) from each magnetic field sensor (32, 33) is equal to the dipole field ($H_{dip}$) emitted by the magnetic object (2) multiplied by a sensitivity factor of the magnetic field sensor (32, 33).

4. The method (1) according to the preceding claim, wherein each calibration electrical signal is equal to the dipole field emitted by the calibration magnetic object at a calibration magnetic field sensor multiplied by a calibration sensitivity factor, the calibration sensitivity factors being equal to the sensitivity factors of both magnetic field sensors (32, 33).

5. The method (1) according to one of the preceding claims, comprising a step (10) of determining the reference curve comprising the following sub-steps of:

- for different passage heights of the calibration magnetic object between two calibration magnetic field sensors:

o determining the dipole magnetic field emitted by the calibration magnetic object and perceived by one of the calibration magnetic field sensors;
o determining the dipole magnetic field emitted by the calibration magnetic object and perceived by the other calibration magnetic field sensor;

- calculating the ratio of the amplitudes of the dipole magnetic fields perceived by the calibration magnetic field sensors as a function of the different passage heights of the calibration magnetic object.

6. The method (1) according to one of the preceding claims, wherein the reception step (11) also comprises a sub-step (11c) of identifying synchronised electrical signals comprising measuring a time difference ($t_{SYN}$) between characteristic signatures of both electrical signals (TOP, BOTTOM), each characteristic signature corresponding to the measurement of the magnetic object (2) by one of the magnetic field sensors (32, 33), synchronisation being identified when the time difference ($t_{SYN}$) is within a predetermined time range ($W_{SYN}$).

7. The method (1) according to one of the two preceding claims, wherein the reception step (11) comprises a sub-step of identifying (11b) characteristic signatures in each of the electrical signals from shape criteria of the electrical signals.

8. The method (1) according to one of the preceding claims, wherein the magnetic object is a biological object marked by means of magnetic beads, said method (1) also comprising a step of calculating (14) the number of magnetic beads associated with the biological object (5) marked from the magnetic moment calculated and the magnetic moment of a single magnetic bead.

9. The method (1) according to one of the preceding claims, wherein the magnetic field sensors (32, 33) are magnetoresistive sensors.

10. A method (4) for counting biological objects (5), comprising the following steps of:

- marking (41) the biological objects (5) by mixing them with a liquid matrix comprising magnetic beads able to attach to receptors carried by each biological object;
- circulating the liquid matrix comprising the biological objects (5) marked in the microfluidic channel (31) of the biochip (3) and determining (42) the magnetic moment ($\mu$) of magnetic objects (2) passing between the magnetic field sensors (32, 33) by means of the method (1) according to one of claims 1 to 9, each magnetic object (2) counting as a biological object (5) marked when the magnetic moment ($\mu$) associated therewith is greater than a threshold magnetic moment (NT).

11. The counting method (4) according to the preceding claim, comprising the step of determining (40) the threshold magnetic moment ($N_T$), said step comprising the following sub-steps of:

   - circulating the liquid matrix without biological objects, referred to as the "negative control", in the microfluidic channel of the biochip (3) and determining the magnetic moment ($\mu$) of magnetic objects (2) passing between the magnetic field sensors by means of the method (1) for determining the magnetic moment according to the invention; and
   - setting the threshold magnetic moment (NT) from a statistical distribution of the magnetic moments determined.

12. A device (6) for determining the magnetic moment ($\mu$) of a magnetic object (2), the magnetic object being a biological object (5) marked by means of magnetic beads or a magnetosome or an aggregate of magnetic beads, comprising:

   - a biochip comprising: a microfluidic channel (31) extending in a plane (P) and having a height (hcan), measured along a direction (Z) referred to as the "normal direction" perpendicular to the plane (P); and two magnetic field sensors (31, 32), disposed on either side of the microfluidic channel (31),
   - a magnet (62) configured to apply a magnetic field (Ho) at the biochip,
   - a system for controlling flow rate of a fluid matrix (65) configured to circulate a liquid matrix comprising the biological objects (5) marked in the microfluidic channel (31) of the biochip (3),
   - an acquisition chain (64) responsible for polarising the sensors (32, 33) and receiving both synchronised electrical signals (TOP, BOTTOM) from said sensors (32, 33), corresponding to the passage of the magnetic object (2) into the microfluidic channel (31) at each magnetic field sensor (32, 33); and comprising a controller configured to:

      o calculate (12) the passage height ($z_2$) of the magnetic object (2), measured along the normal direction (Z), from the ratio of the amplitudes of the synchronised electrical signals (TOP, BOTTOM) and by means of a reference curve ($R(z_b)$) relating the passage height ($z_b$) of a calibration magnetic object in the microfluidic channel (31) to a ratio (R) of the amplitudes of the calibration electrical signals corresponding to the passage of the calibration magnetic object at said passage height ($z_b$), said ratio (R) of the amplitudes of the calibration electrical signals non-linearly increasing as the passage height ($z_b$) of the calibration magnetic object increases until it reaches a maximum value, the ratio of the amplitudes of the synchronised electrical signals (TOP, BOTTOM) being equal to the ratio (R) of the amplitudes of the calibration electrical signals, and
      o calculate (13) the magnetic moment ($\mu$) of the magnetic object (2) from the passage height ($z_2$) determined and one of the synchronised electrical signals.

13. A system for counting biological objects (5), comprising:

   - means for marking (41) the biological objects (5) by mixing them with a liquid matrix comprising magnetic beads able to attach to receptors carried by each biological object;
   - the device for determining the magnetic moment ($\mu$) of a magnetic object according to claim 12, wherein the controller of the acquisition chain is further configured to count a biological object (5) marked when the magnetic moment ($\mu$) associated therewith is greater than a threshold magnetic moment (NT).

14. A computer program comprising instructions which cause the device (6) according to claim 12 to execute the steps of the determination method (1) according to one of claims 1 to 9.

15. A computer program comprising instructions which cause the counting system according to claim 13 to execute the steps of the counting method (4) according to one of claims 10 to 11.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

[Fig. 13]

[Fig. 14]

[Fig. 15]

[Fig. 16]

Billes magnétiques — Anticorps — Albumine — Cellules — Etape 1 — Etape 2 — Etape 3

[Fig. 17]

Bille magnétique — Anticorps (anti-CD138) — Antigène (CD138) — Cellule (NS1)

[Fig. 18]

**RÉFÉRENCES CITÉES DANS LA DESCRIPTION**

**Documents brevets cités dans la description**

- WO 2019238857 A1

- WO 2019238857 A